# EUROPEAN PATENT APPLICATION

(11) **EP 4 151 654 A1**
(43) Date of publication of application: **22.03.2023**
(21) Application number: 21803875.0
(22) Date of filing: 11.05.2021
(51) Int. Cl.: C07K 16/28

(54) **ST2 ANTIGEN BINDING PROTEIN**

(30) Priority: 12.05.2020 CN 202010397572
(71) Applicant: CHIA TAI TIANQING PHARMACEUTICAL GROUP CO., LTD., Lianyungang, Jiangsu 222062 (CN)
(72) Inventor: ZHANG, Zhengping, Lianyungang, Jiangsu 222062 (CN); YING, Shusong, Lianyungang, Jiangsu 222062 (CN); XU, Hongjiang, Lianyungang, Jiangsu 222062 (CN); YANG, Ling, Lianyungang, Jiangsu 222062 (CN); ZHANG, Xiquan, Lianyungang, Jiangsu 222062 (CN); GUO, Jun, Lianyungang, Jiangsu 222062 (CN); SHI, Wei, Lianyungang, Jiangsu 222062 (CN); SONG, Wei, Lianyungang, Jiangsu 222062 (CN); ZHOU, Yunyan, Lianyungang, Jiangsu 222062 (CN)
(74) Representative: Grünecker Patent- und Rechtsanwälte PartG mbB
(86) International application number: PCT/CN2021/093066
(87) International publication number: WO 2021/228091

(57) **Abstract**

Provided is an ST2 antigen binding protein, such as a mouse, human, chimeric or humanized antibody or antigen binding fragment thereof that specifically binds to ST2. Also provided are nucleic acid molecules encoding the above-mentioned antibodies and antigen binding fragments thereof, and an expression vector and host cell for expressing the antibodies or antigen binding fragments thereof. Further provided are methods for preparing and using the antibodies and antigen binding fragments thereof. The methods comprise treating and preventing IL33/ST2-mediated related diseases or conditions.

## Description

### CROSS-REFERENCE TO RELATED APPLICATIONS

The present disclosure claims priority to the Chinese Patent Application No. 202010397572.3 filed on May 12, 2020, the content of which is incorporated herein by reference in its entirety and for all purposes.

### TECHNICAL FIELD

The present disclosure relates to an isolated antibody. In particular, the present disclosure provides mouse, chimeric and humanized monoclonal antibodies or antigen-binding fragments thereof that specifically bind to ST2, and provides nucleic acids, expression vectors and host cells for producing the antibodies or the antigen-binding fragments thereof. The present disclosure further provides polypeptide fusions, multispecific molecules, viral vectors and pharmaceutical compositions that comprise the antibodies or the antigen-binding portions thereof, and provides a method for using the antibodies or the antigen-binding fragments thereof in diagnosis and treatment.

### BACKGROUND

Interleukin 33 (IL33) is a member of the IL-1 cytokine family. It is expressed by endothelial cells or epithelial cells of tissues. When cells are exposed to stimuli such as stress, infection and damage, IL33 is expressed as an "alarmin" in damaged cells and transmits external antigen stimulation signals to the Th2 pathway. The receptor for IL33 consists of two proteins: IL-1 receptor-associated protein (IL-1RL1, ST2) and IL-1 receptor auxiliary protein (IL-1RAP). The binding of IL33 to ST2 can induce signal transduction only in the presence of IL-1RAP, but IL-1RAP cannot bind directly to IL33 or ST2 (Chackerian et al., (2007) J Immunol. 179:2551-2555).

ST2 belongs to the Toll/IL1 receptor family. It has three subtypes: the transmembrane form ST2L, the soluble form sST2, and the variant form ST2V. ST2L mainly mediates the intracellular signal response of IL33. ST2L is mainly expressed on the surfaces of Th2 cells, ILC2 cells, mast cells and Treg cells, and can also be expressed on the surfaces of NK cells, NKT cells, macrophages, eosinophils and basophils.

IL33 binds to ST2L on the cell surface and recruits IL-1RAcP, thereby activating the MyD88/NFκB cell pathway and inducing mast cells, Th2 cells, Treg cells, ILC2 cells, etc. to secrete pro-inflammatory factors such as IL-5, IL-6, IL-13, TNF and INF-y and chemokines such as CCL17, CCL22 and CXCL8 to induce inflammation. IL-33 expression is abnormally elevated in diseases of mucosal tissue inflammation, joint inflammation and chronic skin inflammation, such as allergic rhinitis (Kamekura R et al., (2012) Clin Exp Allergy. 42:218-28), rheumatoid arthritis, ankylosing spondylitis, atopic dermatitis (Savinko T et al., (2012) J Invest Dermatol. 132:1392-1400), and psoriasis; in addition, the dysregulation of IL33/ST2 signaling pathway is closely related to immune-mediated diseases such as asthma, chronic obstructive pulmonary disease (Hacker, Lambers et al., (2009) J Clin Lαb Anal. 23:372-9), bronchitis, inflammatory bowel disease (Beltran CJ et al., (2010) Inflamm Bowel Dis. 16:1097-107), systemic lupus erythematosus, hepatic fibrosis and systemic sclerosis. Gene-level analysis shows that the IL33 and ST2 genes have some single nucleotide polymorphism (SNP) sites, which are related to the number of basophils and are also closely related to the occurrence of atopic dermatitis and asthmatic diseases (Shimizu M et al., (2005) Hum Mol Genet. 14:2919-27; Gudbjartsson DF et al., (2009) Nat Genet. 41:342-7). Therefore, blocking the binding of IL33 to ST2 and inhibiting the IL33/ST2 signaling pathway and the inflammation induced thereby have become important topics in the treatment of inflammatory immune-related diseases.

### BRIEF SUMMARY

The present disclosure provides an ST2 antibody with high affinity, high specificity and high bioactivity suitable for use in treating IL33/ST2-mediated related diseases.

The present disclosure provides isolated antibodies, e.g., mouse, human, chimeric or humanized monoclonal antibodies or antigen-binding fragments thereof that bind to ST2 (e.g., human ST2 and monkey ST2).

The antibodies or the antigen-binding fragments thereof disclosed herein have a variety of uses, including detecting ST2 proteins, and treating and preventing IL33/ST2-mediated related diseases and conditions, and the IL33/ST2-mediated related diseases and conditions include asthma, allergic rhinitis, chronic obstructive pulmonary disease, eosinophilic bronchitis, eosinophilic esophagitis, atopic dermatitis, psoriasis, systemic lupus erythematosus, pemphigoid, rheumatoid arthritis, ankylosing spondylitis, inflammatory bowel disease, pulmonary fibrosis, hepatic fibrosis, systemic sclerosis, sarcoidosis, graft-versus-host disease (GVHD), diabetes, cardiovascular diseases, or combinations thereof.

In one aspect, the present disclosure provides an isolated antibody or an antigen-binding fragment thereof, which comprises heavy chain CDRs, and the heavy chain CDRs comprise heavy chain CDR1, heavy chain CDR2 and heavy chain CDR3, wherein (1) heavy chain CDR1 comprises a sequence set forth in SEQ ID NO: 11 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR2 comprises a sequence set forth in SEQ ID NO: 15 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and heavy chain CDR3 comprises a sequence set forth in SEQ ID NO: 20 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (2) heavy chain CDR1 comprises a sequence set forth in SEQ ID NO: 11 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR2 comprises a sequence set forth in SEQ ID NO: 16 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and heavy chain CDR3 comprises a sequence set forth in SEQ ID NO: 21 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (3) heavy chain CDR1 comprises a sequence set forth in SEQ ID NO: 12 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR2 comprises a sequence set forth in SEQ ID NO: 17 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and heavy chain CDR3 comprises a sequence set forth in SEQ ID NO: 22 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (4) heavy chain CDR1 comprises a sequence set forth in SEQ ID NO: 13 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR2 comprises a sequence set forth in SEQ ID NO: 18 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and heavy chain CDR3 comprises a sequence set forth in SEQ ID NO: 23 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (5) heavy chain CDR1 comprises a sequence set forth in SEQ ID NO: 14 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR2 comprises a sequence set forth in SEQ ID NO: 19 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and heavy chain CDR3 comprises a sequence set forth in SEQ ID NO: 22 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; or (6) heavy chain CDR1 comprises a sequence set forth in SEQ ID NO: 11 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR2 comprises a sequence set forth in SEQ ID NO: 18 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and heavy chain CDR3 comprises a sequence set forth in SEQ ID NO: 24 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto.

In some specific embodiments, the isolated antibody or the antigen-binding fragment thereof binds to ST2, wherein the amino acid sequence of SEQ ID NO: 17 is AIDPETGDTVYX₁X₂KFX₃G, wherein X₁ = N or A, X₂ = Q, E or K, and X₃ = K or Q.

In some specific embodiments, the isolated antibody or the antigen-binding fragment thereof binds to ST2, wherein the amino acid sequence of SEQ ID NO: 17 is AIDPETGDTVYX₁X₂KFX₃G, wherein X₁ = N or A, X₂ = Q, and X₃ = K or Q; X₁ = N or A, X₂ = E, and X₃ = K or Q; or X₁ = N or A, X₂ = K, and X₃ = K or Q.

In some specific embodiments, the isolated antibody or the antigen-binding fragment thereof binds to ST2, wherein the amino acid sequence of SEQ ID NO: 17 is AIDPETGDTVYX₁X₂KFX₃G, wherein X₁ = N, X₂ = Q, and X₃ = K or Q; X₁ = A, X₂ = Q, and X₃ = K or Q; X₁ = N, X₂ = E, and X₃ = K or Q; X₁ = A, X₂ = E, and X₃ = K or Q; X₁ = N, X₂ = K, and X₃ = K or Q; or X₁ = A, X₂ = K, and X₃ = K or Q.

In some specific embodiments, the isolated antibody or the antigen-binding fragment thereof binds to ST2, wherein the amino acid sequence of SEQ ID NO: 17 is AIDPETGDTVYX₁X₂KFX₃G, wherein X₁ = N, X₂ = Q, and X₃ = K; X₁ = A, X₂ = E, and X₃ = Q; or X₁ = A, X₂ = K, and X₃ = K.

In some specific embodiments, the isolated antibody or the antigen-binding fragment thereof binds to ST2, and it is a monoclonal antibody (e.g., a mouse, chimeric or humanized antibody).

In some embodiments, the isolated antibody or the antigen-binding fragment thereof comprises a heavy chain variable region, and the amino acid sequence of the heavy chain variable region comprises a sequence set forth in SEQ ID NO: 35, 37, 39, 41, 43, 45, 71 or 73 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the sequence set forth in SEQ ID NO: 35, 37, 39, 41, 43, 45, 71 or 73.

In some specific embodiments, the isolated antibody or the antigen-binding fragment thereof binds to ST2, and it is a monoclonal antibody (e.g., a mouse, chimeric or humanized antibody).

In some specific embodiments, the amino acids set forth in SEQ ID NO: 71 can be encoded by the nucleic acid set forth in SEQ ID NO: 72; the amino acids set forth in SEQ ID NO: 73 can be encoded by the nucleic acid set forth in SEQ ID NO: 74.

In some embodiments, the isolated antibody or the antigen-binding fragment thereof comprises light chain CDRs, and the light chain CDRs comprise light chain CDR1, light chain CDR2 and light chain CDR3, wherein (1) light chain CDR1 comprises a sequence set forth in SEQ ID NO: 25 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, light chain CDR2 comprises a sequence set forth in SEQ ID NO: 29 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and light chain CDR3 comprises a sequence set forth in SEQ ID NO: 32 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (2) light chain CDR1 comprises a sequence set forth in SEQ ID NO: 26 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, light chain CDR2 comprises a sequence set forth in SEQ ID NO: 30 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and light chain CDR3 comprises a sequence set forth in SEQ ID NO: 34 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (3) light chain CDR1 comprises a sequence set forth in SEQ ID NO: 27 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, light chain CDR2 comprises a sequence set forth in SEQ ID NO: 30 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and light chain CDR3 comprises a sequence set forth in SEQ ID NO: 34 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; or (4) light chain CDR1 comprises a sequence set forth in SEQ ID NO: 28 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, light chain CDR2 comprises a sequence set forth in SEQ ID NO: 31 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and light chain CDR3 comprises a sequence set forth in SEQ ID NO: 33 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto.

In some specific embodiments, the isolated antibody or the antigen-binding fragment thereof binds to ST2, wherein the amino acid sequence of SEQ ID NO: 30 is QX₄SNLAS, X₄ = M or L.

In some specific embodiments, the isolated antibody or the antigen-binding fragment thereof binds to ST2, and it is a monoclonal antibody (e.g., a mouse, chimeric or humanized antibody).

In some embodiments, the isolated antibody or the antigen-binding fragment thereof comprises a light chain variable region, and the amino acid sequence of the light chain variable region comprises a sequence set forth in SEQ ID NO: 36, 38, 40, 42, 44, 46 or 79 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the sequence set forth in SEQ ID NO: 36, 38, 40, 42, 44, 46 or 79.

In some specific embodiments, the isolated antibody or the antigen-binding fragment thereof binds to ST2, and it is a monoclonal antibody (e.g., a mouse, chimeric or humanized antibody).

In some specific embodiments, the amino acids set forth in SEQ ID NO: 79 can be encoded by the nucleic acid set forth in SEQ ID NO: 80.

In some embodiments, the isolated antibody or the antigen-binding fragment thereof comprises heavy chain CDR1, heavy chain CDR2 and heavy chain CDR3, and light chain CDR1, light chain CDR2 and light chain CDR3, wherein (1) heavy chain CDR1 comprises a sequence set forth in SEQ ID NO: 11 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR2 comprises a sequence set forth in SEQ ID NO: 15 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR3 comprises a sequence set forth in SEQ ID NO: 20 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, light chain CDR1 comprises a sequence set forth in SEQ ID NO: 25 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, light chain CDR2 comprises a sequence set forth in SEQ ID NO: 29 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and light chain CDR3 comprises a sequence set forth in SEQ ID NO: 32 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (2) heavy chain CDR1 comprises a sequence set forth in SEQ ID NO: 11 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR2 comprises a sequence set forth in SEQ ID NO: 16 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR3 comprises a sequence set forth in SEQ ID NO: 21 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, light chain CDR1 comprises a sequence set forth in SEQ ID NO: 26 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, light chain CDR2 comprises a sequence set forth in SEQ ID NO: 30 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and light chain CDR3 comprises a sequence set forth in SEQ ID NO: 34 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (3) heavy chain CDR1 comprises a sequence set forth in SEQ ID NO: 12 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR2 comprises a sequence set forth in SEQ ID NO: 17 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR3 comprises a sequence set forth in SEQ ID NO: 22 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, light chain CDR1 comprises a sequence set forth in SEQ ID NO: 27 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, light chain CDR2 comprises a sequence set forth in SEQ ID NO: 30 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and light chain CDR3 comprises a sequence set forth in SEQ ID NO: 34 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (4) heavy chain CDR1 comprises a sequence set forth in SEQ ID NO: 13 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR2 comprises a sequence set forth in SEQ ID NO: 18 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR3 comprises a sequence set forth in SEQ ID NO: 23 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, light chain CDR1 comprises a sequence set forth in SEQ ID NO: 27 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, light chain CDR2 comprises a sequence set forth in SEQ ID NO: 30 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and light chain CDR3 comprises a sequence set forth in SEQ ID NO: 34 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (5) heavy chain CDR1 comprises a sequence set forth in SEQ ID NO: 14 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR2 comprises a sequence set forth in SEQ ID NO: 19 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR3 comprises a sequence set forth in SEQ ID NO: 22 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, light chain CDR1 comprises a sequence set forth in SEQ ID NO: 27 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, light chain CDR2 comprises a sequence set forth in SEQ ID NO: 30 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and light chain CDR3 comprises a sequence set forth in SEQ ID NO: 34 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; or (6) heavy chain CDR1 comprises a sequence set forth in SEQ ID NO: 11 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR2 comprises a sequence set forth in SEQ ID NO: 18 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, heavy chain CDR3 comprises a sequence set forth in SEQ ID NO: 24 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, light chain CDR1 comprises a sequence set forth in SEQ ID NO: 28 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, light chain CDR2 comprises a sequence set forth in SEQ ID NO: 31 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and light chain CDR3 comprises a sequence set forth in SEQ ID NO: 33 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto.

In some specific embodiments, the isolated antibody or the antigen-binding fragment thereof binds to ST2, wherein the amino acid sequence of SEQ ID NO: 17 is AIDPETGDTVYX₁X₂KFX₃G, wherein X₁ = N or A, X₂ = Q, E or K, and X₃ = K or Q; and the amino acid sequence of SEQ ID NO: 30 is QX₄SNLAS, wherein X₄ = M or L.

In some specific embodiments, the isolated antibody or the antigen-binding fragment thereof binds to ST2, wherein the amino acid sequence of SEQ ID NO: 17 is AIDPETGDTVYX₁X₂KFX₃G, wherein X₁ = N or A, X₂ = Q, and X₃ = K or Q; X₁ = N or A, X₂ = E, and X₃ = K or Q; X₁ = N or A, X₂ = K, and X₃ = K or Q; and the amino acid sequence of SEQ ID NO: 30 is QX₄SNLAS, wherein X₄ = M or L.

In some specific embodiments, the isolated antibody or the antigen-binding fragment thereof binds to ST2, wherein the amino acid sequence of SEQ ID NO: 17 is AIDPETGDTVYX₁X₂KFX₃G, wherein X₁ = N, X₂ = Q, and X₃ = K or Q; X₁ = A, X₂ = Q, and X₃ = K or Q; X₁ = N, X₂ = E, and X₃ = K or Q; X₁ = A, X₂ = E, and X₃ = K or Q; X₁ = N, X₂ = K, and X₃ =K or Q; or X₁ = A, X₂ = K, and X₃ =K or Q; and the amino acid sequence of SEQ ID NO: 30 is QX₄SNLAS, wherein X₄ = M or L.

In some specific embodiments, the isolated antibody or the antigen-binding fragment thereof binds to ST2, wherein the amino acid sequence of SEQ ID NO: 17 is AIDPETGDTVYX₁X₂KFX₃G, wherein X₁ = N, X₂ = Q, and X₃ = K; X₁ = A, X₂ = E, and X₃ = Q; or X₁ = A, X₂ = K, and X₃ = K; and the amino acid sequence of SEQ ID NO: 30 is QX₄SNLAS, wherein X₄ = M or L.

In some specific embodiments, the isolated antibody or the antigen-binding fragment thereof binds to ST2, and it is a monoclonal antibody (e.g., a mouse, chimeric or humanized antibody).

In some embodiments, the isolated antibody or the antigen-binding fragment thereof comprises a heavy chain variable region and a light chain variable region, wherein (1) the heavy chain variable region comprises a sequence set forth in SEQ ID NO: 35 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and the light chain variable region comprises a sequence set forth in SEQ ID NO: 36 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (2) the heavy chain variable region comprises a sequence set forth in SEQ ID NO: 37 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and the light chain variable region comprises a sequence set forth in SEQ ID NO: 38 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (3) the heavy chain variable region comprises a sequence set forth in SEQ ID NO: 39 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and the light chain variable region comprises a sequence set forth in SEQ ID NO: 40 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (4) the heavy chain variable region comprises a sequence set forth in SEQ ID NO: 41 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and the light chain variable region comprises a sequence set forth in SEQ ID NO: 42 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (5) the heavy chain variable region comprises a sequence set forth in SEQ ID NO: 43 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and the light chain variable region comprises a sequence set forth in SEQ ID NO: 44 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (6) the heavy chain variable region comprises a sequence set forth in SEQ ID NO: 45 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and the light chain variable region comprises a sequence set forth in SEQ ID NO: 46 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (7) the heavy chain variable region comprises a sequence set forth in SEQ ID NO: 71 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and the light chain variable region comprises a sequence set forth in SEQ ID NO: 79 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; or (8) the heavy chain variable region comprises a sequence set forth in SEQ ID NO: 73 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and the light chain variable region comprises a sequence set forth in SEQ ID NO: 79 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto.

In some specific embodiments, the isolated antibody or the antigen-binding fragment thereof binds to ST2, and it is a monoclonal antibody (e.g., a mouse, chimeric or humanized antibody).

In some embodiments, the isolated antibody or the antigen-binding fragment thereof comprises a heavy chain, and the amino acid sequence of the heavy chain comprises a sequence set forth in SEQ ID NO: 47, 51, 55, 59, 63, 67, 75 or 77 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the sequence set forth in SEQ ID NO: 47, 51, 55, 59, 63, 67, 75 or 77.

In some specific embodiments, the isolated antibody or the antigen-binding fragment thereof binds to ST2, and it is a monoclonal antibody (e.g., a mouse, chimeric or humanized antibody).

In some specific embodiments, the amino acids set forth in the aforementioned SEQ ID NOs: 47, 51, 55, 59, 63, 67, 75 and 77 can be encoded by the nucleic acids set forth in SEQ ID NOs: 48, 52, 56, 60, 64, 68, 76 and 78, respectively.

In some embodiments, the isolated antibody or the antigen-binding fragment thereof comprises a light chain, and the amino acid sequence of the light chain comprises a sequence set forth in SEQ ID NO: 49, 53, 57, 61, 65, 69 or 81 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the sequence set forth in SEQ ID NO: 49, 53, 57, 61, 65, 69 or 81.

In some specific embodiments, the isolated antibody or the antigen-binding fragment thereof binds to ST2, and it is a monoclonal antibody (e.g., a mouse, chimeric or humanized antibody).

In some specific embodiments, the amino acids set forth in the aforementioned SEQ ID NOs: 49, 53, 57, 61, 65, 69 and 81 can be encoded by the nucleic acids set forth in SEQ ID NOs: 50, 54, 58, 62, 66, 70 and 82, respectively.

In some embodiments, the isolated antibody or the antigen-binding fragment thereof comprises a heavy chain and a light chain, wherein (1) the heavy chain comprises a sequence set forth in SEQ ID NO: 47 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and the light chain comprises a sequence set forth in SEQ ID NO: 49 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (2) the heavy chain comprises a sequence set forth in SEQ ID NO: 51 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and the light chain comprises a sequence set forth in SEQ ID NO: 53 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (3) the heavy chain comprises a sequence set forth in SEQ ID NO: 55 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and the light chain comprises a sequence set forth in SEQ ID NO: 57 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (4) the heavy chain comprises a sequence set forth in SEQ ID NO: 59 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and the light chain comprises a sequence set forth in SEQ ID NO: 61 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (5) the heavy chain comprises a sequence set forth in SEQ ID NO: 63 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and the light chain comprises a sequence set forth in SEQ ID NO: 65 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (6) the heavy chain comprises a sequence set forth in SEQ ID NO: 67 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and the light chain comprises a sequence set forth in SEQ ID NO: 69 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; (7) the heavy chain comprises a sequence set forth in SEQ ID NO: 75 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and the light chain comprises a sequence set forth in SEQ ID NO: 81 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; or (8) the heavy chain comprises a sequence set forth in SEQ ID NO: 77 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto, and the light chain comprises a sequence set forth in SEQ ID NO: 81 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto.

In some specific embodiments, the isolated antibody or the antigen-binding fragment thereof binds to ST2, and it is a monoclonal antibody (e.g., a mouse, chimeric or humanized antibody).

In some embodiments, the isolated antibody (e.g., a mouse, chimeric or humanized antibody) or the antigen-binding fragment thereof comprises a heavy chain and a light chain, the heavy chain comprises a heavy chain variable region and a heavy chain constant region, and the light chain comprises a light chain variable region and a light chain constant region, wherein the heavy chain variable region and the light chain variable region comprise the amino acid sequences described above; the heavy chain constant region comprises a human IgG1, IgG2 or IgG4 constant region, preferably a human IgG2 constant region; the light chain constant region comprises a human κ constant region or a human λ constant region, wherein the antibody or the antigen-binding fragment thereof binds to ST2.

In some embodiments, the antibody disclosed herein comprises or consists of two heavy chains and two light chains, the heavy chains and light chains being linked to each other by disulfide bonds, wherein each heavy chain comprises the heavy chain constant region, the heavy chain variable region or CDR sequences described above, and each light chain comprises the light chain constant region, the light chain variable region or CDR sequences described above, wherein the C-terminus of the heavy chain variable region is linked to the N-terminus of the heavy chain constant region, and the C-terminus of the light chain variable region is linked to the N-terminus of the light chain constant region. The antibody disclosed herein can be, e.g., a full-length antibody of the IgG1, IgG2 or IgG4 isotype. In another embodiment, the antibody disclosed herein can be a single-chain antibody (scFv), or an antibody fragment, such as an Fab, an F(ab')2 fragment, an Fd fragment, an Fv fragment, a dAb, or an isolated CDR.

In one aspect, the present disclosure also provides a polypeptide fusion comprising the antibody or the antigen-binding fragment thereof disclosed herein and other functional molecules, wherein the other functional molecules may be peptides, proteins or non-proteins. The polypeptide fusion has a function of binding to ST2, and one or more other functions. In one aspect, the present disclosure also provides a multispecific molecule comprising the antibody or the antigen-binding fragment thereof disclosed herein, and at least one other functional portion with specificity that is different from that of the antibody or the antigen-binding fragment, wherein the other functional portion may be another peptide or protein. The multispecific molecule is able to bind to ST2 and to at least one other disease-associated protein, e.g., IgE. In another aspect, the ST2 antibody or the antigen-binding fragment thereof disclosed herein can also be encoded or carried by a viral vector.

In one aspect, the present disclosure also provides a pharmaceutical composition comprising the antibody or the antigen-binding fragment thereof disclosed herein, or comprising the multispecific molecule, the polypeptide fusion, or the viral vector disclosed herein, and a pharmaceutically acceptable excipient, diluent or carrier.

In one aspect, the present disclosure also provides an isolated nucleic acid molecule encoding the antibody or the antigen-binding fragment thereof disclosed herein, an expression vector comprising such nucleic acid molecule, and a host cell comprising such expression vector.

In one aspect, the present disclosure also provides a method for preparing an ST2 antibody or an antigen-binding fragment thereof, which comprises the following steps: (i) expressing the antibody or the antigen-binding fragment thereof in a host cell, and (ii) isolating the antibody or the antigen-binding fragment thereof from the host cell or its cell culture.

In another aspect, the present disclosure provides a method for determining an ST2 expression level in a sample from a subject, which comprises a step of contacting the sample with the antibody or the antigen-binding fragment thereof disclosed herein under such conditions that the antibody or the antigen-binding fragment thereof is allowed to bind to ST2 (or form a complex with ST2).

In another aspect, the present disclosure provides a method for treating or alleviating IL33/ST2-mediated related diseases and conditions in a subject in need, the IL33/ST2-mediated related diseases and conditions including but not limited to asthma, allergic rhinitis, chronic obstructive pulmonary disease, eosinophilic bronchitis, eosinophilic esophagitis, atopic dermatitis, psoriasis, systemic lupus erythematosus, pemphigoid, rheumatoid arthritis, ankylosing spondylitis, inflammatory bowel disease, pulmonary fibrosis, hepatic fibrosis, systemic sclerosis, sarcoidosis, graft-versus-host disease (GVHD), diabetes, cardiovascular diseases, or combinations thereof, the method comprising administering to the subject a therapeutically effective amount of the antibody or the antigen-binding fragment thereof or the pharmaceutical composition thereof disclosed herein. In some embodiments, the method comprises administering to the subject a therapeutically effective amount of the nucleic acid molecule encoding the antibody or the antigen-binding fragment thereof disclosed herein. In some embodiments, the method comprises administering to the subject a therapeutically effective amount of the polypeptide fusion, the multispecific molecule, the viral vector, or the pharmaceutical composition thereof disclosed herein. In some embodiments, the multispecific molecule is able to bind to ST2 and to at least another disease-associated protein, e.g., TSLP, IgE, IL4, IL13 or IL-5. In some embodiments, at least one other antibody, e.g., a TSLP antibody, a TSLPR antibody, an IL4 antibody, an IL4R antibody, or an IgE antibody, can be administered together with the antibody or the antigen-binding fragment thereof disclosed herein. In some embodiments, at least one additional drug, e.g., an anti-asthma drug, an anti-chronic obstructive pulmonary disease drug, an anti-ulcerative colitis drug, an anti-atopic dermatitis drug, or an anti-psoriasis drug, can be administered together with the antibody or the antigen-binding fragment thereof disclosed herein.

Other features and advantages of the present disclosure will be apparent from the following detailed description and examples, which should not be construed as limiting. The content of all documents, Genbank entries, patents and published patent applications cited herein is expressly incorporated herein by reference.

### BRIEF DESCRIPTION OF THE DRAWINGS

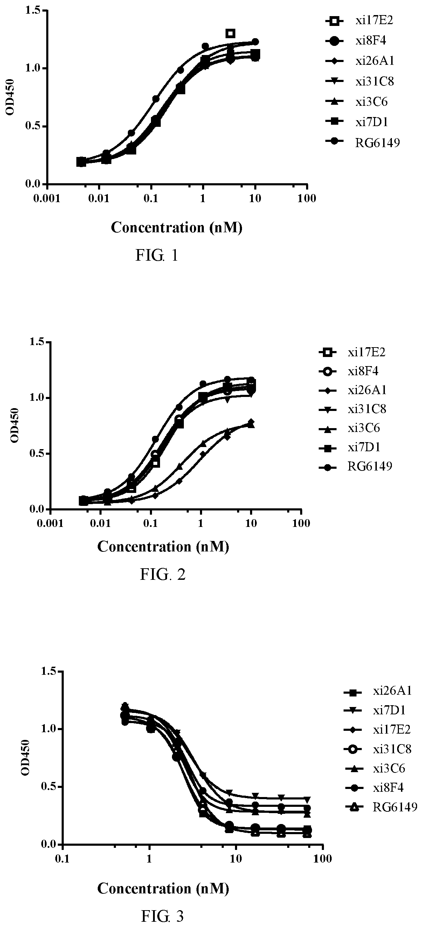

FIG. 1: the binding of chimeric anti-ST2 antibodies to hST2 detected by ELISA over a range of concentrations. FIG. 1 shows the binding of chimeric antibodies xi17E2, xi8F4, xi26A1, xi31C8, xi3C6 and xi7D1 to hST2, with the binding of RG6149 shown as a control.
FIG. 2: the binding of chimeric anti-ST2 antibodies to cyno-ST2 detected by ELISA over a range of concentrations. FIG. 2 shows the binding of chimeric antibodies xi17E2, xi8F4, xi26A1, xi31C8, xi3C6 and xi7D1 to cyno-ST2, with the binding of RG6149 shown as a control.
FIG. 3: the blocking of IL33/ST2 binding by chimeric anti-ST2 antibodies detected by ELISA over a range of antibody concentrations. FIG. 3 shows the blocking of IL33/ST2 binding by chimeric antibodies xi26A1, xi7D1, xi17E2, xi31C8, xi3C6 and xi8F4, with the blocking by RG6149 shown as a control.

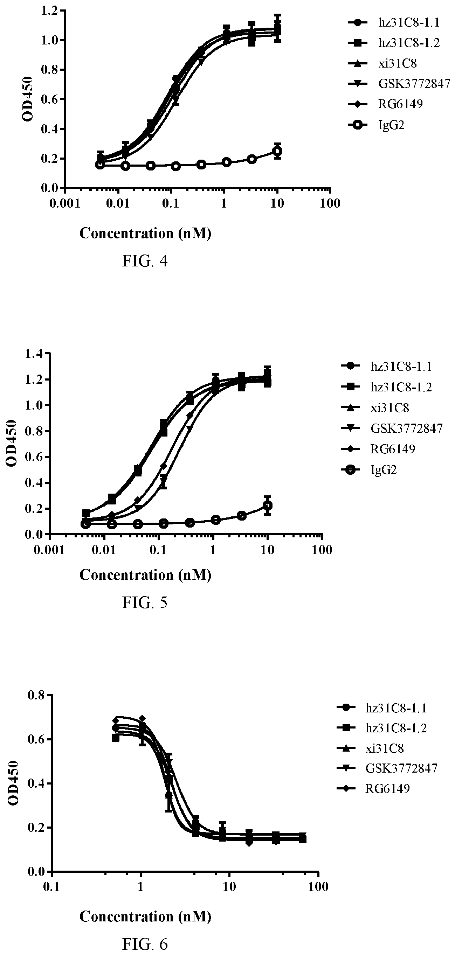

FIG. 4: the binding of humanized anti-ST2 antibodies to hST2 detected by ELISA over a range of concentrations. FIG. 4 shows the binding of humanized antibodies hz31C8-1.1 and hz31C8-1.2 to hST2, with the binding of chimeric antibodies xi31C8, RG6149 and GSK3772847 shown as controls and that of IgG2 as a negative control.
FIG. 5: the binding of humanized anti-ST2 antibodies to cyno-ST2 detected by ELISA over a range of concentrations. FIG. 5 shows the binding of humanized antibodies hz31C8-1.1 and hz31C8-1.2 to cyno-ST2, with the binding of chimeric antibodies xi31C8, RG6149 and GSK3772847 shown as controls and that of IgG2 as a negative control.
FIG. 6: the blocking of IL33/ST2 binding by humanized anti-ST2 antibodies detected by ELISA over a range of antibody concentrations. FIG. 6 shows the blocking of IL33/ST2 binding by humanized antibodies hz31C8-1.1 and hz31C8-1.2, with the blocking by chimeric antibodies xi31C8, RG6149 and GSK3772847 shown as controls.

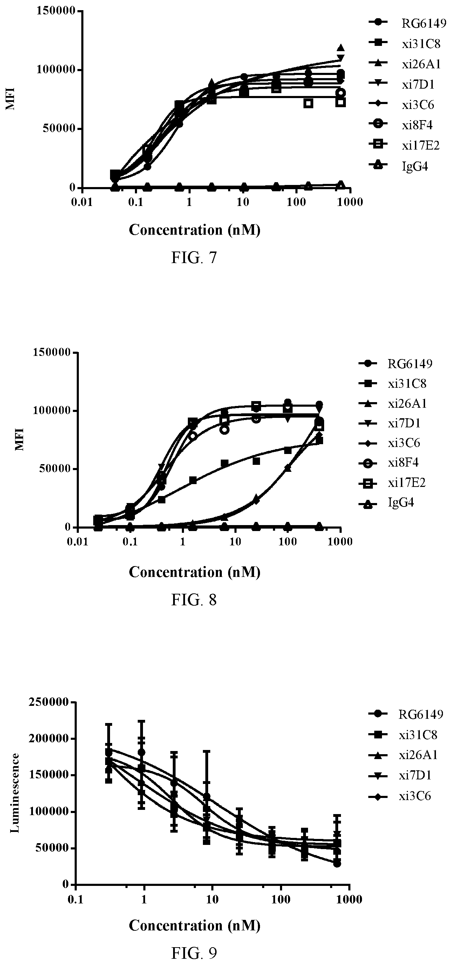

FIG. 7: the binding of chimeric anti-ST2 antibodies to hST2-overexpressing cells detected by FACS over a range of antibody concentrations. FIG. 7 shows the binding of chimeric antibodies xi31C8, xi26A1, xi7D1, xi3C6, xi8F4 and xi17E2 to hST2-overexpressing HEK293T-hST2-NFxB-Luciferase cells, with the binding of RG6149 shown as a control and that of IgG4 as a negative control.
FIG. 8: the binding of chimeric anti-ST2 antibodies to cyno-ST2-overexpressing cells detected by FACS over a range of antibody concentrations. FIG. 8 shows the binding of chimeric antibodies xi31C8, xi26A1, xi7D1, xi3C6, xi8F4 and xi17E2 to cyno-ST2-overexpressing HEK293T-Cyno-ST2-NFxB-Luciferase cells, with the binding of RG6149 shown as a control and that of IgG4 as a negative control.
FIG. 9: the blocking of IL33/ST2 binding by chimeric anti-ST2 antibodies detected through cell viability assays over a range of antibody concentrations. FIG. 9 shows the blocking of interaction between IL33 protein and hST2-expressing HEK293T-hST2-NFxB-Luciferase cells by chimeric antibodies xi31C8, xi26A1, xi7D1 and xi3C6, with the blocking by RG6149 shown as a control.

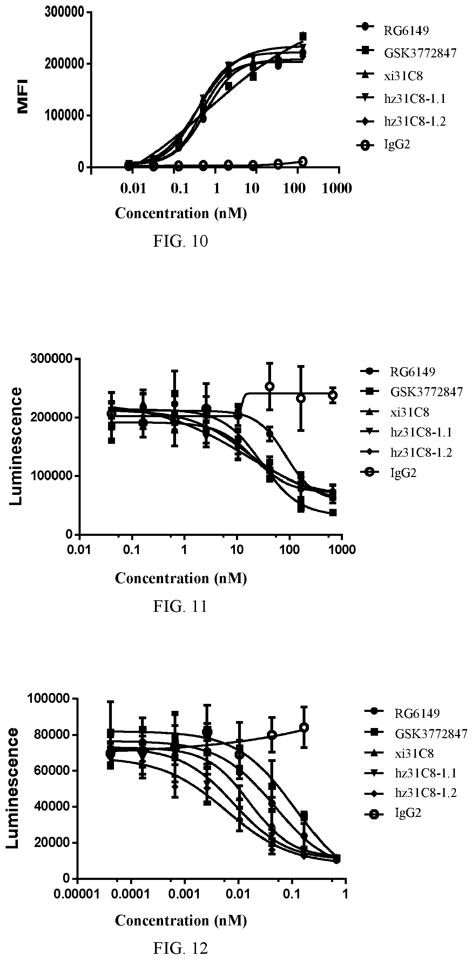

FIG. 10: the binding of humanized anti-ST2 antibodies to hST2-overexpressing cells detected by FACS over a range of antibody concentrations. FIG. 10 shows the binding of humanized antibodies hz31C8-1.1 and hz31C8-1.2 to hST2-overexpressing HEK293T-hST2-NFxB-Luciferase cells, with the binding of chimeric antibodies xi31C8, RG6149 and GSK3772847 shown as controls and that of IgG2 as a negative control.
FIG. 11: the blocking of IL33/ST2 binding by humanized anti-ST2 antibodies detected through cell viability assays over a range of antibody concentrations. FIG. 11 shows the blocking of interaction between IL33 protein and hST2-expressing HEK293T-hST2-NFxB-Luciferase cells by humanized antibodies hz31C8-1.1 and hz31C8-1.2, with the blocking by chimeric antibodies xi31C8, RG6149 and GSK3772847 shown as controls and that by IgG2 as a negative control.
FIG. 12: the blocking of IL33/ST2 binding by humanized anti-ST2 antibodies detected through cell viability assays over a range of antibody concentrations. FIG. 12 shows the blocking of interaction between IL33 protein and hST2-expressing KU812-NFxB-Luciferasae cells by humanized antibodies hz31C8-1.1 and hz31C8-1.2, with the blocking by chimeric antibodies xi31C8, RG6149 and GSK3772847 shown as controls and that by IgG2 as a negative control.

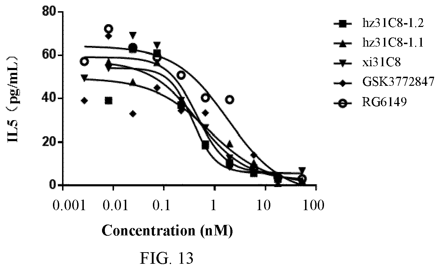

FIG. 13: the blocking of IL33/ST2-induced IL5 secretion on CD4⁺ T cells by humanized anti-ST2 antibodies detected through cell viability assays over a range of antibody concentrations. FIG. 13 shows the blocking of IL33/ST2-induced IL5 secretion on CD4⁺ T cells by humanized antibodies hz31C8-1.1 and hz31C8-1.2, with the blocking by chimeric antibodies xi31C8, RG6149 and GSK3772847 shown as controls.

### SUMMARY

It should be understood that the terminology used herein is for the purpose of describing specific embodiments only and is not intended to be limiting. Unless otherwise defined, all of the technical and scientific terms used herein have the same meaning as commonly understood by those of ordinary skill in the art to which the present disclosure belongs.

"ST2" comprises variants, homologs, orthologs and paralogs of ST2. For example, in some embodiments, an antibody specific for human ST2 protein may cross-react with an ST2 protein of another species (e.g., monkey) in certain circumstances. In other embodiments, an antibody specific for human ST2 protein may be completely specific for human ST2 protein and not cross-react with proteins of other species or other types, or may cross-react with ST2 proteins from certain other species but not alle other species.

The terms "human ST2" and "hST2" and the like are interchangeable in the present disclosure and refer to a protein with the amino acid sequence of human ST2, e.g., the human ST2 amino acid sequence set forth in SEQ ID NO: 1, and the protein consists of several domains: a leader sequence corresponding to amino acids 1-18, an extracellular domain corresponding to amino acids 19-331, a transmembrane domain corresponding to amino acids 332-350, and an intracellular domain corresponding to amino acids 351-556. The terms "monkey ST2" and "cyno-ST2" and the like are interchangeable in the present disclosure and refer to a protein with the amino acid sequence of monkey ST2, e.g. the monkey ST2 amino acid sequence set forth in SEQ ID NO: 10.

The "antibody" disclosed herein includes a full-length antibody and any antigen-binding fragments (i.e., "antigen-binding portion") or single chains thereof. The full-length antibody is a glycoprotein comprising two heavy (H) chains and two light (L) chains, the heavy cahins and light chains being linked by disulfide bonds. Each heavy chain consists of a heavy chain variable region (VH) and a heavy chain constant region. The heavy chain constant region consists of three domains, i.e., CH1, CH2 and CH3. Each light chain consists of a light chain variable region (VL) and a light chain constant region. The light chain constant region consists of one domain CL. The VH and VL regions can be further divided into hypervariable regions, i.e., complementarity determining regions (CDRs), and framework regions (FRs) with more conserved sequences. Each VH and VL consists of three CDRs and four FRs arranged from amino-terminus to carboxy-terminus as follows: FR1, CDR1, FR2, CDR2, FR3, CDR3, FR4. The variable regions of the heavy chains and light chains comprise binding domains that interact with antigens. The constant regions of the antibody can mediate the binding of immunoglobulins to host tissues or factors, including various cells of the immune system (e.g., effector cells) and the first component of the classical complement system (C1q).

The "antigen-binding fragment" or "antibody-binding portion" of the antibody refers to one or more fragments of the antibody that retain the ability to specifically binding to antigens (e.g., ST2 proteins). It has been demonstrated that the antigen-binding function of an antibody can be performed by fragments of a full-length antibody. Examples encompassed within the term "antigen-binding portion/fragment" of the antibody include: (i) a Fab fragment: a monovalent fragment consisting of the V_{L},V_{H}, CL and CH1 domains; (ii) a F(ab')2 fragment, a bivalent fragment comprising two Fab fragments linked by a disulfide bridge in the hinge region; (iii) a Fd fragment consisting of the VH and CH1 domains; (iv) a Fv fragment consisting of the VL and VH domains of a single arm of the antibody; (v) a dAb fragment consisting of the VH domain (Ward et al., Nature. 341:544-546 (1989)); (vi) an isolated complementarity determining region (CDR); and (vii) a nanobody, a heavy chain variable regions comprising a single variable domain and two constant domains. Furthermore, although the two domains of the Fv fragment, VL and VH, are encoded by different genes, they can be joined, using recombinant methods, by a synthetic linker to form a single protein chain in which the VL and VH pair to form a monovalent molecule (referred to as single-chain Fv (scFv), see, e.g., Bird et al., Science. 242:423-426 (1988); Huston et al., Proc. Natl. Acad. Sci. 85:5879-5883 (1988)). Such single-chain antibodies are also encompassed within the term antigen-binding portion/fragment. These antibody fragments can be obtained using conventional techniques known to those skilled in the art, and the fragments can be subjected to functional screening using the same method as full-length antibodies.

An "isolated antibody" refers to an antibody that is substantially free of other antibodies with different antigenic specificities (e.g., an isolated antibody that specifically binds to an ST2 protein is substantially free of antibodies that specifically bind to antigens other than the ST2 protein). However, an isolated antibody that specifically binds to human ST2 protein may cross-bind to other antigens (e.g., ST2 proteins from other species). Furthermore, the isolated antibody is substantially free of other cellular components and/or chemical substances.

A "mouse antibody" or "murine antibody" refers to an antibody in which the framework region and CDRs in the variable region are derived from mouse germline immunoglobulin sequences. Furthermore, if the antibody comprises a constant region, the constant region is also derived from mouse germline immunoglobulin sequences. The mouse antibody disclosed herein may comprise amino acid residues not encoded by mouse germline immunoglobulin sequences (e.g., mutations introduced by *in vitro* random mutation or point mutation or by *in vivo* somatic mutation), but a "mouse antibody" does not include antibodies in which CDR sequences derived from other mammalian species have been grafted onto the mouse framework sequence.

A "chimeric antibody" refers to an antibody formed by combining genetic materials of human sourse and those of non-human sourse. More generally, a chimeric antibody refers to an antibody comprising genetic materials from one species and those from another species. In the present disclosure, chimeric antibodies are also denoted as "Xi".

A "humanized antibody" refers to an antibody from a non-human species whose protein sequence has been modified to increase its similarity to a naturally occurring human antibody. In the present disclosure, humanized antibodies are also denoted as "hz".

"Isotype" refers to the antibody class that is encoded by the heavy chain constant region genes (e.g., IgM or IgG1).

"Antigen-recognizing antibody" and "antigen-specific antibody" are used interchangeably in the present disclosure with the term "antibody that specifically binds to an antigen".

An antibody that "specifically binds to human ST2" refers to one that binds to human ST2 protein (and possibly other ST2 proteins from non-human species) but does not substantially bind to non-ST2 proteins. Preferably, the antibody binds to human ST2 with "high affinity", i.e., a KD of 5.0 × 10⁻⁸ M or less, 1.0 × 10⁻⁸ M or less, preferably 5.0 × 10⁻⁹ M or less, 1.0 × 10⁻⁹ M or less, and more preferably 5.0 × 10⁻¹⁰ M or less, 1.0 × 10⁻¹⁰ M or less.

The term "not substantially bind to" a protein or cell refers to not binding to the protein or cell, or not binding to it with high affinity, i.e., binding to the protein or cell with a KD of 1.0 × 10⁻⁶ M or higher, preferably 1.0 × 10⁻⁵ M or higher, 1.0 × 10⁻⁴ M or higher, and more preferably 1.0 × 10⁻³ M or higher, 1.0 × 10⁻² M or higher.

For IgG, the term "high affinity" refers to a KD of 1.0 × 10⁻⁶ M or less, 1.0 × 10⁻⁷ M or less, preferably 1.0 × 10⁻⁸ M or less, 5.0 × 10⁻⁹ M or less, and more preferably 1.0 × 10⁻⁹ M or less. However, for other antibody isotypes, "high affinity" binding may be different. For example, "high affinity" binding of IgM isotype refers to a KD of 10⁻⁶ M or less, preferably 10⁻⁷ M or less, and more preferably 10⁻⁸ M or less.

"Identity" refers to the similarity between two nucleic acid sequences or between two polypeptides. The identity of the sequences disclosed herein is at least 80%, 85%, 90% or 95%, preferably at least 95%. Non-limiting examples include: 80%, 81%, 82%, 83%, 84%, 85%, 86%, 87%, 88%, 89%, 90%, 91%, 92%, 93%, 94%, 95%, 96%, 97%, 98%, 99%, or 100%. Sequence comparison and percent identity determination between two sequences can be performed using the BLASTN/BLASTP algorithm on the National Center For Biotechnology Institute with default settings.

The term "EC₅₀", also known as half maximal effective concentration, refers to the concentration of antibody that can cause 50% of the maximum effect after a specified exposure time.

The term "IC₅₀", also known as half maximal inhibitory concentration, refers to the concentration of an antibody that inhibits a specific biological or biochemical function by 50% relative to the absence of the antibody.

The terms "inhibit" and "block" are used interchangeably and include partial and complete inhibition/blocking. In some embodiments, the ST2 antibody inhibits the binding of IL33/ST2 by at least about 50%, e.g., at least about 60%, 70%, 80%, 90%, 95%, 99% or 100%.

The term "subject" includes any human or non-human animal. The term "non-human animal" includes all vertebrates, e.g. mammals and non-mammals, preferably mammals, e.g., non-human primates, sheep, dogs, cats, cows and horses.

The term "therapeutically effective amount" refers to an amount sufficient to prevent or ameliorate a disease or condition and/or reduce the severity of the disease or condition, preferably an amount that causes a reduction in the severity of the symptoms of the disease or an increase in the frequency and duration of asymptomatic phases, or is able to prevent the damage or inability caused by the disease. The therapeutically effective amount is related to the disease to be treated, wherein the actual effective amount can be readily determined by those skilled in the art.

The use of singular forms includes plural forms unless otherwise specified. The word "a" or "an" refers to "at least one" unless otherwise specified. The phrase "at least one" means the same as "one or more", and "and/or" is used to mean "and" or "or", unless otherwise stated.

Aspects of the present disclosure are described in more detail below.

The anti-ST2 antibody specifically binds to ST2 and blocks the IL33/ST2 interaction, among other beneficial

### functional features.

The ST2 antibody or the antigen-binding fragment thereof disclosed herein specifically binds to human ST2 with high affinity. The ST2 antibody or the antigen-binding fragment thereof disclosed herein specifically binds to ST2 (e.g., human ST2 and monkey ST2) and blocks IL33/ST2 binding and signaling thereof. The ST2 antibody or the antigen-binding fragment thereof disclosed herein blocks IL5 secretion induced by IL33/ST2 on CD4⁺ T cells. The ST2 antibody or the antigen-binding fragment thereof disclosed herein does not substantially bind to other receptors of the IL-1R family, e.g., IL1R1, IL1R2, IL1R3, IL1R7, IL1R8, and IL1R9. The ST2 antibody or the antigen-binding fragment thereof disclosed herein has good physical stability (e.g., thermostability). The ST2 antibody or the antigen-binding fragment thereof disclosed herein with a long half-life *in vivo.*

Preferably, the ST2 antibody disclosed herein is a monoclonal antibody. Furthermore, the antibody may be, for example, a mouse, chimeric or humanized monoclonal antibody.

### ST2 monoclonal antibody

Preferably, the ST2 antibody or the antigen-binding fragment thereof disclosed herein is an antibody having the structural and chemical properties described below. The ST2 antibody or the antigen-binding fragment thereof comprises heavy chain CDRs and light chain CDRs, wherein exemplary heavy chain CDR sequences and light chain CDR sequences are provided below in Table 1 and Table 2; the ST2 antibody or the antigen-binding fragment thereof comprises heavy chain variable regions and light chain variable regions, wherein exemplary heavy chain variable regions and light chain variable regions are provided below in Table 3. Some antibodies sharing the same CDRs, and some antibodies sharing the same VHs or VLs. The heavy chain constant region of the antibody can be a human IgG2 heavy chain constant region, and the light chain constant region of the antibody can be a human κ light chain constant region. These antibodies may also comprise a mouse IgG1 or IgG4 heavy chain constant region and/or a mouse κ light chain constant region.

The heavy chain CDRs in Table 1 and the light chain CDRs in Table 2 are defined by the Kabat numbering system. However, as is well known in the art, the CDRs may also be determined by other numbering systems such as the Chothia, IMGT, AbM or Contact numbering system/method based on the heavy/light chain variable region sequence.

**Table 1. Amino acid sequence listing of heavy chain CDRs**

| Antibody | HV-CDR1 | SEQ ID NO | HV-CDR2 | SEQ ID NO | HV-CDR3 | SEQ ID NO |
|---|---|---|---|---|---|---|
| 7D1 | DYEMH | 11 | TIDPETGDTAYNQKFKG | 15 | YSYTNSPYGMD Y | 20 |
| 3C6 | DYEMH | 11 | AIDPETGDTAYSQNFKG | 16 | SATDYYFAS | 21 |
| 31C8 | DSEMH | 12 | AIDPETGDTVYX₁X₂KFX₃G (X₁ =N or A; X₂ = Q, E or K; X₃ = K or Q) | 17 | STTDYYFAY | 22 |
| 26A1 | DSEIH | 13 | AIDPETGDTAYNQKFKG | 18 | STTDCYFAY | 23 |
| 8F4 | DYEMQ | 14 | AFDPETGDTAYNQKFKG | 19 | STTDYYFAY | 22 |
| 17E2 | DYEMH | 11 | AIDPETGDTAYNQKFKG | 18 | VDSNYDYFDY | 24 |

**Table 2. Amino acid sequence listing of light chain CDRs**

| Antibody | LV-CDR1 | SEQ ID NO | LV-CDR2 | SEQ ID NO | LV-CDR3 | SEQ ID NO |
|---|---|---|---|---|---|---|
| 7D1 | KASQSVSNDVA | 25 | YASNRYT | 29 | QQDYSSPYT | 32 |
| 3C6 | RSSKSLLHSNGITYLY | 26 | QX₄SNLAS (X₄ = M or L) | 30 | AQNLELPFT | 34 |
| 31C8 | RSSKSLLHSNGIIYLY | 27 | QX₄SNLAS (X₄ = M or L) | 30 | AQNLELPFT | 34 |
| 26A1 | RSSKSLLHSNGIIYLY | 27 | QX₄SNLAS (X₄ = M or L) | 30 | AQNLELPFT | 34 |
| 8F4 | RSSKSLLHSNGIIYLY | 27 | QX₄SNLAS (X₄ = M or L) | 30 | AQNLELPFT | 34 |
| 17E2 | RASSSVSYMH | 28 | DTSNLAS | 31 | QQWSSNPLT | 33 |

**Table 3. Amino acid sequence listing of heavy/light chain variable regions (CDRs are underlined in sequence)**

| Antibody | | | SEQ ID NO |
|---|---|---|---|
| 7D1 mouse | HV | QVQLQQSGAELVRPGASVTLSCKASGYTFTDYEMHWVKQTPVHGLDWI GTIDPETGDTAYNQKFKGRATLTADKSSSTAYMELSSLTSEDSAVYYCTRY SYTNSPYGMDYWGQGTSVTVSS | 35 |
| 7D1 mouse | LV | DVVMTQTPKFLLVSAGDRVTITCKASQSVSNDVAWYQQKPGQSPKLLIYY ASNRYTGVPDRFTGSGYGTDFTFTINTVQAEDLAVYFCQQDYSSPYTFGG GTKLEIK | 36 |
| 3C6 mouse | HV | QVQLQQSGAEVVRPGASVTLSCKASDYTITDYEMHWVKQTPVHGLEWI GAIDPETGDTAYSQNFKGKATLTADESSSTAYMELSSLTSEDSAVYYCSRS ATDYYFASWGQGTTLTVSS | 37 |
| 3C6 mouse | LV | DIVMTQAAISNPVTLGTSASMSCRSSKSLLHSNGITYLYWYLQKPGHSPQ LLIYQMSNLASGVPDRFSSSGSGTDFTLKISRVEAEDVGVYYCAQNLELPF TFGSGTKLEIK | 38 |
| 31C8 mouse | HV | QVQLQQSGPELVRPGASVTLSCKASGYIFIDSEMHWVKQTPVHGLEWIG AIDPETGDTVYNQKFKGKATLTADKSSSTASMELSSLTSEDSAVYYCTGST TDYYFAYWGQGTTLTVSS | 39 |
| 31C8 mouse | LV | DIVMTQAAFSNPVTLGTSASISCRSSKSLLHSNGIIYLYWYLQKPGQSPQL LIYQMSNLASGVPDRFSSSGSGTDFTLRISRVEAEDVGVYYCAQNLELPFT FGSGTKLEIK | 40 |
| 26A1 mouse | HV | QVQLQQSGAELVRPGASVTLSCKASDYIFTDSEIHWVKQTLVHGLEWIGA IDPETGDTAYNQKFKGKATLTADKSSSTAYMELSSLTSEGSAVYYCSGSTT DCYFAYWGQGTTLTVSS | 41 |
| 26A1 mouse | LV | DIVMTQAAFSNPVTLGTSASISCRSSKSLLHSNGIIYLYWYLQKPGQSPQL LIYQMSNLASGVPDRFSSSGSGTDFTLRISRVEAEDVGVYYCAQNLELPFT FGSGTKLEIK | 42 |
| 8F4 mouse | HV | QVQLQQSGAELVRPGASVTLSCKASGHIFTDYEMQWVKQTPVHGLEWIG AFDPETGDTAYNQKFKGKATLTADKSSSTAYMELSSLTSEDSAVYYCSGST TDYYFAYWGQGTSLTVSS | 43 |
| 8F4 mouse | LV | DVVMTQTAFSNPVTLGTSASISCRSSKSLLHSNGIIYLYWYLQKPGQSPQL LIYQMSNLASGVPDRFSSSGSGTDFTLRISRVEAEDVGVYYCAQNLELPFT FGSGTKLEIK | 44 |
| 17E2 mouse | HV | QVQLQQSGAELVRPGASVTLSCKASGYTFTDYEMHWVKQTPVLGLEWI GAIDPETGDTAYNQKFKGKATLTADKSSSTAYMELSSLASEDSAVYYCIRV DSNYDYFDYWGQDTTLTVSS | 45 |
| 17E2 mouse | LV | DIVLTQSPPILSASPGEKVTMTCRASSSVSYMHWYQQKPGSSPKPWIYDTS NLASGVPARFSGSGSGTSYSLTISRVEAEDAATYYCQQWSSNPLTFGAGTK LELK | 46 |
| Humanized 31C8-1.1 | HV | EVQLVQSGAEVKKPGATVKISCKASGYIFIDSEMHWVQQAPGKGLEWMG AIDPETGDTVYAEKFQGRVTITADKSSSTAYMELSSLRSEDTAVYYCTGST TDYYFAYWGQGTLVTVSS | 71 |
| Humanized 31C8-1.1 | LV | DIQMTQSPSSLSASVGDRVTITCRSSKSLLHSNGIIYLYWYQQKPGKAPKL LIYQLSNLASGVPSRFSSSGSGTDFTLTISSLQPEDFATYYCAQNLELPFTFG QGTKVEIK | 79 |
| Humanized 31C8-1.2 | HV | QVQLVQSGAKVKKPGATVKISCKASGYIFIDSEMHWVQQAPGKGLEWM GAIDPETGDTVYAKKFKGRVTITADKSSSTAYMELSSLRSEDTAVYYCTGS TTDYYFAYWGQGTLVTVSS | 73 |
| Humanized 31C8-1.2 | LV | DIQMTQSPSSLSASVGDRVTITCRSSKSLLHSNGIIYLYWYQQKPGKAPKL LIYQLSNLASGVPSRFSSSGSGTDFTLTISSLQPEDFATYYCAQNLELPFTFG QGTKVEIK | 79 |

**Table 4. Amino acid sequence listing of heavy chains/light chains (constant regions are underlined)**

| Antibody | | | SEQ ID NO |
|---|---|---|---|
| Chimeric 7D1 | H | QVQLQQSGAELVRPGASVTLSCKASGYTFTDYEMHWVKQTPVHGL DWIGTIDPETGDTAYNQKFKGRATLTADKSSSTAYMELSSLTSEDSAV YYCTRYSYTNSPYGMDYWGQGTSVTVSSASTKGPSVFPLAPCSRST SESTAALGCLVKDYFPEPVTVSWNSGALTSGVHTFPAVLQSSGLYSLS SVVTVPSSNFGTQTYTCNVDHKPSNTKVDKTVERKCCVECPPCPAPP VAGPSVFLFPPKPKDTLMISRTPEVTCVVVDVSHEDPEVQFNWYVD GVEVHNAKTKPREEQFNSTFRVVSVLTVVHQDWLNGKEYKCKVSN KGLPAPIEKTISKTKGQPREPQVYTLPPSREEMTKNQVSLTCLVKGFY PSDISVEWESNGQPENNYKTTPPMLDSDGSFFLYSKLTVDKSRWQQ GNVFSCSVMHEALHNHYTQKSLSLSPGK | 47 |
| Chimeric 7D1 | L | DVVMTQTPKFLLVSAGDRVTITCKASQSVSNDVAWYQQKPGQSPKL LIYYASNRYTGVPDRFTGSGYGTDFTFTINTVQAEDLAVYFCQQDYS SPYTFGGGTKLEIKRTVAAPSVFIFPPSDEQLKSGTASVVCLLNNFYP REAKVQWKVDNALQSGNSQESVTEQDSKDSTYSLSSTLTLSKADYE KHKVYACEVTHQGLSSPVTKSFNRGEC | 49 |
| Chimeric 3C6 | H | QVQLQQSGAEVVRPGASVTLSCKASDYTITDYEMHWVKQTPVHGL EWIGAIDPETGDTAYSQNFKGKATLTADESSSTAYMELSSLTSEDSAV YYCSRSATDYYFASWGQGTTLTVSSASTKGPSVFPLAPCSRSTSESTA ALGCLVKDYFPEPVTVSWNSGALTSGVHTFPAVLQSSGLYSLSSVVT VPSSNFGTQTYTCNVDHKPSNTKVDKTVERKCCVECPPCPAPPVAGP SVFLFPPKPKDTLMISRTPEVTCVVVDVSHEDPEVQFNWYVDGVEV HNAKTKPREEQFNSTFRVVSVLTVVHQDWLNGKEYKCKVSNKGLP APIEKTISKTKGQPREPQVYTLPPSREEMTKNQVSLTCLVKGFYPSDI SVEWESNGQPENNYKTTPPMLDSDGSFFLYSKLTVDKSRWQQGNVF SCSVMHEALHNHYTQKSLSLSPGK | 51 |
| Chimeric 3C6 | L | DIVMTQAAISNPVTLGTSASMSCRSSKSLLHSNGITYLYWYLQKPGH SPQLLIYQMSNLASGVPDRFSSSGSGTDFTLKISRVEAEDVGVYYCA QNLELPFTFGSGTKLEIKRTVAAPSVFIFPPSDEQLKSGTASVVCLLNN FYPREAKVQWKVDNALQSGNSQESVTEQDSKDSTYSLSSTLTLSKA DYEKHKVYACEVTHQGLSSPVTKSFNRGEC | 53 |
| Chimeric | H | QVQLQQSGAELVRPGASVTLSCKASDYIFTDSEIHWVKQTLVHGLE | 55 |
| 26A1 | | WIGAIDPETGDTAYNQKFKGKATLTADKSSSTAYMELSSLTSEGSAVY YCSGSTTDCYFAYWGQGTTLTVSSASTKGPSVFPLAPCSRSTSESTAA LGCLVKDYFPEPVTVSWNSGALTSGVHTFPAVLQSSGLYSLSSVVTV PSSNFGTQTYTCNVDHKPSNTKVDKTVERKCCVECPPCPAPPVAGPS VFLFPPKPKDTLMISRTPEVTCVVVDVSHEDPEVQFNWYVDGVEVH NAKTKPREEQFNSTFRVVSVLTVVHQDWLNGKEYKCKVSNKGLPAP IEKTISKTKGQPREPQVYTLPPSREEMTKNQVSLTCLVKGFYPSDISV EWESNGQPENNYKTTPPMLDSDGSFFLYSKLTVDKSRWQQGNVFSC SVMHEALHNHYTQKSLSLSPGK | |
| Chimeric 26A1 | L | DIVMTQAAFSNPVTLGTSASISCRSSKSLLHSNGIIYLYWYLQKPGQS PQLLIYQMSNLASGVPDRFSSSGSGTDFTLRISRVEAEDVGVYYCAQ NLELPFTFGSGTKLEIKRTVAAPSVFIFPPSDEQLKSGTASVVCLLNNF YPREAKVQWKVDNALQSGNSQESVTEQDSKDSTYSLSSTLTLSKAD YEKHKVYACEVTHQGLSSPVTKSFNRGEC | 57 |
| Chimeric 8F4 | H | QVQLQQSGAELVRPGASVTLSCKASGHIFTDYEMQWVKQTPVHGL EWIGAFDPETGDTAYNQKFKGKATLTADKSSSTAYMELSSLTSEDSA VYYCSGSTTDYYFAYWGQGTSLTVSSASTKGPSVFPLAPCSRSTSEST AALGCLVKDYFPEPVTVSWNSGALTSGVHTFPAVLQSSGLYSLSSVV TVPSSNFGTQTYTCNVDHKPSNTKVDKTVERKCCVECPPCPAPPVAG PSVFLFPPKPKDTLMISRTPEVTCVVVDVSHEDPEVQFNWYVDGVE VHNAKTKPREEQFNSTFRVVSVLTVVHQDWLNGKEYKCKVSNKGL PAPIEKTISKTKGQPREPQVYTLPPSREEMTKNQVSLTCLVKGFYPSDI SVEWESNGQPENNYKTTPPMLDSDGSFFLYSKLTVDKSRWQQGNVF SCSVMHEALHNHYTQKSLSLSPGK | 59 |
| Chimeric 8F4 | L | DVVMTQTAFSNPVTLGTSASISCRSSKSLLHSNGIIYLYWYLQKPGQS PQLLIYQMSNLASGVPDRFSSSGSGTDFTLRISRVEAEDVGVYYCAQ NLELPFTFGSGTKLEIKRTVAAPSVFIFPPSDEQLKSGTASVVCLLNNF YPREAKVQWKVDNALQSGNSQESVTEQDSKDSTYSLSSTLTLSKAD YEKHKVYACEVTHQGLSSPVTKSFNRGEC | 61 |
| Chimeric 17E2 | H | QVQLQQSGAELVRPGASVTLSCKASGYTFTDYEMHWVKQTPVLGL EWIGAIDPETGDTAYNQKFKGKATLTADKSSSTAYMELSSLASEDSAV YYCIRVDSNYDYFDYWGQDTTLTVSSASTKGPSVFPLAPCSRSTSEST AALGCLVKDYFPEPVTVSWNSGALTSGVHTFPAVLQSSGLYSLSSVV TVPSSNFGTQTYTCNVDHKPSNTKVDKTVERKCCVECPPCPAPPVAG PSVFLFPPKPKDTLMISRTPEVTCVVVDVSHEDPEVQFNWYVDGVE VHNAKTKPREEQFNSTFRVVSVLTVVHQDWLNGKEYKCKVSNKGL PAPIEKTISKTKGQPREPQVYTLPPSREEMTKNQVSLTCLVKGFYPSDI SVEWESNGQPENNYKTTPPMLDSDGSFFLYSKLTVDKSRWQQGNVF SCSVMHEALHNHYTQKSLSLSPGK | 63 |
| Chimeric 17E2 | L | DIVLTQSPPILSASPGEKVTMTCRASSSVSYMHWYQQKPGSSPKPWI YDTSNLASGVPARFSGSGSGTSYSLTISRVEAEDAATYYCQQWSSNP LTFGAGTKLELKRTVAAPSVFIFPPSDEQLKSGTASVVCLLNNFYPRE AKVQWKVDNALQSGNSQESVTEQDSKDSTYSLSSTLTLSKADYEKH KVYACEVTHQGLSSPVTKSFNRGEC | 65 |
| Chimeric 31C8 | H | QVQLQQSGPELVRPGASVTLSCKASGYIFIDSEMHWVKQTPVHGLE WIGAIDPETGDTVYNQKFKGKATLTADKSSSTASMELSSLTSEDSAVY YCTGSTTDYYFAYWGQGTTLTVSSASTKGPSVFPLAPCSRSTSESTA ALGCLVKDYFPEPVTVSWNSGALTSGVHTFPAVLQSSGLYSLSSVVT | 67 |
| | | VPSSNFGTQTYTCNVDHKPSNTKVDKTVERKCCVECPPCPAPPVAGP SVFLFPPKPKDTLMISRTPEVTCVVVDVSHEDPEVQFNWYVDGVEV HNAKTKPREEQFNSTFRVVSVLTVVHQDWLNGKEYKCKVSNKGLP APIEKTISKTKGQPREPQVYTLPPSREEMTKNQVSLTCLVKGFYPSDI SVEWESNGQPENNYKTTPPMLDSDGSFFLYSKLTVDKSRWQQGNVF SCSVMHEALHNHYTQKSLSLSPGK | |
| Chimeric 31C8 | L | DIVMTQAAFSNPVTLGTSASISCRSSKSLLHSNGIIYLYWYLQKPGQS PQLLIYQMSNLASGVPDRFSSSGSGTDFTLRISRVEAEDVGVYYCAQ NLELPFTFGSGTKLEIKRTVAAPSVFIFPPSDEQLKSGTASVVCLLNNF YPREAKVQWKVDNALQSGNSQESVTEQDSKDSTYSLSSTLTLSKAD YEKHKVYACEVTHQGLSSPVTKSFNRGEC | 69 |
| Humanized 31C8-1.1 | H | EVQLVQSGAEVKKPGATVKISCKASGYIFDSEMHWVQQAPGKGLE WMGAIDPETGDTVYAEKFQGRVTITADKSSSTAYMELSSLRSEDTAV YYCTGSTTDYYFAYWGQGTLVTVSSASTKGPSVFPLAPCSRSTSEST AALGCLVKDYFPEPVTVSWNSGALTSGVHTFPAVLQSSGLYSLSSVV TVPSSNFGTQTYTCNVDHKPSNTKVDKTVERKCCVECPPCPAPPVAG PSVFLFPPKPKDTLMISRTPEVTCVVVDVSHEDPEVQFNWYVDGVE VHNAKTKPREEQFNSTFRVVSVLTVVHQDWLNGKEYKCKVSNKGL PAPIEKTISKTKGQPREPQVYTLPPSREEMTKNQVSLTCLVKGFYPSDI SVEWESNGQPENNYKTTPPMLDSDGSFFLYSKLTVDKSRWQQGNVF SCSVMHEALHNHYTQKSLSLSPGK | 75 |
| Humanized 31C8-1.1 | L | DIQMTQSPSSLSASVGDRVTITCRSSKSLLHSNGIIYLYWYQQKPGKA PKLLIYQLSNLASGVPSRFSSSGSGTDFTLTISSLQPEDFATYYCAQNL ELPFTFGQGTKVEIKRTVAAPSVFIFPPSDEQLKSGTASVVCLLNNFYP REAKVQWKVDNALQSGNSQESVTEQDSKDSTYSLSSTLTLSKADYE KHKVYACEVTHQGLSSPVTKSFNRGEC | 81 |
| Humanized 31C8-1.2 | H | QVQLVQSGAKVKKPGATVKISCKASGYIFIDSEMHWVQQAPGKGLE WMGAIDPETGDTVYAKKFKGRVTITADKSSSTAYMELSSLRSEDTAV YYCTGSTTDYYFAYWGQGTLVTVSSASTKGPSVFPLAPCSRSTSEST AALGCLVKDYFPEPVTVSWNSGALTSGVHTFPAVLQSSGLYSLSSVV TVPSSNFGTQTYTCNVDHKPSNTKVDKTVERKCCVECPPCPAPPVAG PSVFLFPPKPKDTLMISRTPEVTCVVVDVSHEDPEVQFNWYVDGVE VHNAKTKPREEQFNSTFRVVSVLTVVHQDWLNGKEYKCKVSNKGL PAPIEKTISKTKGQPREPQVYTLPPSREEMTKNQVSLTCLVKGFYPSDI SVEWESNGQPENNYKTTPPMLDSDGSFFLYSKLTVDKSRWQQGNVF SCSVMHEALHNHYTQKSLSLSPGK | 77 |
| Humanized 31C8-1.2 | L | DIQMTQSPSSLSASVGDRVTITCRSSKSLLHSNGIIYLYWYQQKPGKA PKLLIYQLSNLASGVPSRFSSSGSGTDFTLTISSLQPEDFATYYCAQNL ELPFTFGQGTKVEIKRTVAAPSVFIFPPSDEQLKSGTASVVCLLNNFYP REAKVQWKVDNALQSGNSQESVTEQDSKDSTYSLSSTLTLSKADYE KHKVYACEVTHQGLSSPVTKSFNRGEC | 81 |

The VH and/or VL sequences (or CDR sequences) of other anti-ST2 antibodies which bind to human ST2 can be "mixed and matched" with the VH and/or VL sequences (or CDR sequences) of the antibody disclosed herein. Preferably, when VH and VL chains (or CDRs within such chains) are mixed and matched, a VH sequence from a particular VH/VL pairing can be substituted with a structurally similar VH sequence. Likewise, preferably a VL sequence from a particular VH/VL pairing can be substituted with a structurally similar VL sequence.

Therefore, in one embodiment, the antibody or the antigen-binding fragment thereof disclosed herein comprises:
(a) a heavy chain variable region comprising an amino acid sequence listed in Table 3; and
(b) a light chain variable region comprising an amino acid sequence listed in Table 3, or the VL of another ST2 antibody, wherein the antibody specifically binds to human ST2.

In another embodiment, the antibody or the antigen-binding fragment thereof disclosed herein comprises:
(a) heavy chain CDR1, CDR2 and CDR3 listed in Table 1; and
(b) light chain CDR1, CDR2 and CDR3 listed in Table 2, or the CDRs of another ST2 antibody, wherein the antibody specifically binds to human ST2.

In another embodiment, the antibody or the antigen-binding fragment thereof disclosed herein comprises the heavy chain CDR2 of the ST2 antibody disclosed herein and the CDRs of other antibodies that bind to human ST2, e.g., the heavy chain CDR1 and/or CDR3, and/or the light chain CDR1, CDR2 and/or CDR3 of another ST2 antibody.

Furthermore, it is well known in the art that the CDR3 domain, independently from the CDR1 and/or CDR2 domain(s), alone can determine the antibody's binding specificity for identical antigens, and that multiple antibodies with the same binding specificity can be predicted based on the CDR3 sequence. See, e.g., Klimka et al., British J .of Cancer. 83(2):252-260 (2000); Beiboer et al., J .Mol .Biol. 296:833-849 (2000); Rader et al., Proc. Natl. Acad. Sci. U.S.A. 95:8910-8915 (1998); Barbas et al., J. Am. Chem. Soc. 116:2161-2162 (1994); Barbas et al., Proc. Natl. Acad. Sci. U.S.A. 92:2529-2533 (1995); Ditzel et al., J .Immunol. 157:739-749 (1996); Berezov et al., BIAjournαl 8: Scientific Review 8 (2001); Igarashi et al., J .Biochem (Tokyo). 117:452-7 (1995); Bourgeois et al., J .Virol. 72:807-10 (1998); Levi et al., Proc. Natl. Acad. Sci. U.S.A. 90:4374-8 (1993); Polymenis and Stoller, J. Immunol.152:5218-5329 (1994) and Xu and Davis, Immunity. 13:37-45 (2000); and U.S. Pat. Nos. 6,951,646; 6,914,128; 6,090,382; 6,818,216; 6,156,313; 6,827,925; 5,833,943; 5,762,905 and 5,760,185. These references are all incorporated herein by reference in their entirety.

In another embodiment, the antibody or the antigen-binding fragment thereof disclosed herein comprises the heavy chain CDR2 of the ST2 antibody disclosed herein and at least the heavy chain and/or light chain CDR3 of the ST2 antibody disclosed herein, or the heavy chain and/or light chain CDR3 of another ST2 antibody, wherein the antibody specifically binds to human ST2. Preferably, these antibodies (a) compete for binding to ST2; (b) retain functional characteristics; (c) bind to the same epitope; and/or (d) have similar binding affinity as the ST2 antibody disclosed herein. In another embodiment, the antibody or the antigen-binding fragment thereof disclosed herein may further comprise the light chain CDR2 of the ST2 antibody disclosed herein, or the light chain CDR2 of another ST2 antibody, wherein the antibody specifically binds to human ST2. In another embodiment, the antibody or the antigen-binding fragment thereof disclosed herein may further comprise the heavy and/or light chain CDR1 of the ST2 antibody disclosed herein, or the heavy and/or light chain CDR1 of another ST2 antibody, wherein the antibody specifically binds to human ST2.

### Conservative modification

In another embodiment, the antibody or the antigen-binding fragment thereof disclosed herein comprises the CDR1, CDR2 and CDR3 sequences of a heavy chain variable region and/or a light chain variable region comprising one or more conservative modifications relative to the ST2 antibody disclosed herein. It is understood in the art that some conservative sequence modifications do not eliminate the antigen-binding ability. See, e.g., Brummell et al., Biochem 32:1180-8 (1993); de Wildt et al., Prot. Eng. 10:835-41 (1997); Komissarov et al., J. Biol. Chem. 272:26864-26870 (1997); Hall et al., J. Immunol. 149:1605-12 (1992); Kelley and O'Connell Biochem. 32:6862-35 (1993); Adib-Conquy et al., Int. Immunol. 10: 341-6 (1998); and Beers et al., Clin. Can. Res. 6:2835-43 (2000).

Therefore, in one embodiment, the antibody comprises a heavy chain variable region and/or a light chain variable region each comprising CDR1, CDR2 and CDR3, wherein:
(a) the CDR1 sequence of the heavy chain variable region comprises the sequences listed in Table 1, and/or conservative modifications thereof; and/or
(a) the CDR2 sequence of the heavy chain variable region comprises the sequences listed in Table 1, and/or conservative modifications thereof; and/or
(c) the CDR3 sequence of the heavy chain variable region comprises the sequences listed in Table 1, and/or conservative modifications thereof; and/or
(d) the CDR1 and/or CDR2 and/or CDR3 sequences of the light chain variable region comprise the sequences listed in Table 2; and/or conservative modifications thereof; and
(e) the antibody specifically binds to human ST2.

The antibody disclosed herein has one or more of the following functional properties described above, e.g., having high affinity for human ST2, and blocking IL33/ST2 binding and signaling thereof.

In multiple embodiments, the antibody may be a mouse, chimeric or humanized antibody or an antigen-binding fragment thereof.

The term "conservative sequence modification" as used herein refers to an amino acid modification that does not significantly affect or alter the binding property of the antibody. Such conservative modifications include amino acid substitutions, additions and deletions. Modifications can be introduced into the antibody disclosed herein using standard techniques known in the art, such as point mutation and PCR-mediated mutation. Conservative amino acid substitutions are those in which an amino acid residue is replaced with an amino acid residue having a similar side chain. Families of amino acid residues having similar side chains are known in the art. These amino acid residue families include amino acids with basic side chains (e.g., lysine, arginine, and histidine), amino acids with acidic side chains (e.g., aspartic acid, and glutamic acid), amino acids with uncharged polar side chains (e.g., glycine, asparagine, glutamine, serine, threonine, tyrosine, cysteine, and tryptophan), amino acids with non-polar side chains (e.g., alanine, valine, leucine, isoleucine, proline, phenylalanine, and methionine), amino acids with β-branched side chains (e.g., threonine, valine, and isoleucine), and amino acids with aromatic side chains (e.g., tyrosine, phenylalanine, tryptophan, and histidine). Therefore, one or more amino acid residues in the CDRs of the antibody disclosed herein can be substituted with other amino acid residues of the same side chain family, and the resulting antibody can be tested for retained functions (i.e., the functions described above) using the functional tests described in the present disclosure.

### Engineered and modified antibody

The antibody disclosed herein can be engineered to produce modified antibodies using antibodies having one or more VH/VL sequences of the ST2 antibody disclosed herein as starting materials. One or more residues within one or both of the variable regions (i.e., VH and/or VL) (e.g., within one or more CDRs and/or one or more framework regions) of the antibody can be genetically modified. Furthermore, or alternatively, residues in the constant region of the antibody can be engineered, for example, to alter the effector function of the antibody.

In certain embodiments, CDR grafting can be used to genetically modify the variable regions of the antibody. The antibody interacts with the target antigen mainly through amino acid residues in the six heavy chain and light chain complementarity determining regions (CDRs). Therefore, the amino acid sequences within the CDRs of each antibody is more diverse than the sequences outside the CDRs. Since the CDR sequences are responsible for the major antibody-antigen interactions, expression vectors in which the CDR sequences of particular naturally occurring antibodies are grafted into the framework sequences of different antibodies with different properties can be constructed to express recombinant antibodies simulating the properties of the particular naturally occurring antibodies (Riechmann et al., Nature. 332:323-327 (1998); Jones et al., Nature. 321:522-525 (1986); Queen et al., Proc. Natl. Acad. U.S.A. 86:10029-10033 (1989); and U.S. Pat. Nos. 5,225,539; 5,530,101; 5,585,089; 5,693,762 and 6,180,370).

Accordingly, another embodiment of the present disclosure relates to an isolated monoclonal antibody or an antigen-binding fragment thereof, which comprises a heavy chain variable region comprising CDR1, CDR2 and CDR3 of the sequences described above in the present disclosure and/or a light chain variable region comprising CDR1, CDR2 and CDR3 of the sequences described above in the present disclosure. Although these antibodies comprise the CDR sequences of the VH and VL of the monoclonal antibody disclosed herein, they may comprise different framework sequences.

Such framework sequences can be found in public DNA databases or public references including germline antibody gene sequences. For example, germline DNA sequences for human heavy chain variable region and light chain variable region genes can be found in the Vbase human germline sequence database (www.mrc-cpe.cam.ac.uk/Vbase) and Kabat et al, (1991), supra; Tomlinson et al., J. Mol. Biol. 227:776-798 (1992); and Cox et al., Eur. J. Immunol. 24:827-836 (1994). In another embodiment, germline DNA sequences for human heavy chain variable region and light chain variable region genes can be found in the Genbank database.

Antibody protein sequences were compared to protein sequence databases using one of the sequence similarity search methods of Gapped BLAST (Altschul et al, (1997), supra) that are well known to those skilled in the art.

The framework sequences of the antibodies disclosed herein are preferably those that are structurally similar to the framework sequences used for the antibodies disclosed herein. The VH CDR1, CDR2 and CDR3 sequences can be grafted into a framework region that comprises the same sequence as the germline immunoglobulin gene from which the framework sequences were derived, or the CDR sequences can be grafted into a framework region that comprises one or more mutations compared to the germline sequence. For example, in some cases, it may be beneficial to mutate residues in the framework region; such mutations can maintain or enhance the antigen-binding ability of the antibody (see, e.g., U.S. Pat. Nos. 5,530,101; 5,585,089; 5,693,762 and 6,180,370). Another type of variable region modification is to mutate amino acid residues within CDR1, CDR2 and/or CDR3 of the VH and/or VL to improve one or more properties (e.g., affinity and physicochemical properties) of the target antibody. Mutations can be introduced by site-directed mutagenesis or PCR-mediated mutagenesis, and the effect of the mutations on antibody binding or other functional properties can be assessed through *in vitro* or *in vivo* assays known in the art. Preferably, conservative modifications known in the art are introduced. The conservative modifications may be amino acid substitutions, additions or deletions, preferably substitutions. Furthermore, typically no more than one, two, three, four or five residues within each CDR are altered. Furthermore, in another embodiment, the present disclosure provides an isolated ST2 monoclonal antibody or an antigen-binding fragment thereof, which comprises a heavy chain variable region and a light chain variable region comprising: (a) VH CDR1 comprising the sequence of the present disclosure or an amino acid sequence comprising 1, 2, 3, 4 or 5 amino acid substitutions, deletions or additions; (b) VH CDR2 comprising the sequence of the present disclosure or an amino acid sequence comprising 1, 2, 3, 4 or 5 amino acid substitutions, deletions or additions; (c) VH CDR3 comprising the sequence of the present disclosure or an amino acid sequence comprising 1, 2, 3, 4 or 5 amino acid substitutions, deletions or additions; (d) VL CDR1 comprising the sequence of the present disclosure or an amino acid sequence comprising 1, 2, 3, 4 or 5 amino acid substitutions, deletions or additions; (e) VL CDR2 comprising the sequence of the present disclosure or an amino acid sequence comprising 1, 2, 3, 4 or 5 amino acid substitutions, deletions or additions; and (f) VL CDR3 comprising the sequence of the present disclosure or an amino acid sequence comprising 1, 2, 3, 4 or 5 amino acid substitutions, deletions or additions.

The genetically engineered antibodies disclosed herein include those antibodies in which the framework region residues of the VH and/or VL were modified to improve the antibodies' properties. In general, such framework region modifications can reduce the immunogenicity of the antibody. For example, one or more framework residues are "back mutated" to the corresponding germline sequence. More specifically, antibodies undergoing somatic mutation may contain framework region residues that differ from the resulting antibody germline sequence. These residues can be identified by comparing the antibody framework sequence to the germline sequence of the resulting antibody.

Another type of framework modification involves mutating one or more residues of the framework region or even one or more CDRs to remove T cell epitopes, thereby reducing the immunogenicity that an antibody may produce. This method is also known as "deimmunization" and is described in more detail in U.S. patent publication No. 20030153043.

Furthermore, in addition to the framework region or CDR modifications, another type of modification, e.g., modifications to the Fc region of the antibody disclosed herein by genetic engineering, is often used to alter one or more functional properties of the antibody, such as serum half-life, complement fixation, Fc receptor binding, and/or antigen-dependent cytotoxicity. Furthermore, the antibody disclosed herein may also be chemically modified (e.g., to be linked with one or more chemical functional groups), or modified to alter its glycosylation, to alter one or more functional properties of the antibody.

In one embodiment, the CH1-hinge region is modified, for example, by increasing or decreasing the number of cysteine residues in the hinge region. This method is described in detail in U.S. Pat. No. 5,677,425. Altering the number of cysteine residues in the CH1-hinge region can, for example, facilitate the assembly of the light chain and the heavy chain or improve or lower the stability of the antibody.

In another embodiment, the Fc-hinge region of the antibody is mutated to increase or decrease the biological half-life of the antibody. More specifically, one or more amino acid mutations are introduced into the CH2-CH3 region of the Fc-hinge region such that the antibody has reduced staphylococcal protein A (SpA) binding compared to the antibody with the natural Fc-hinge domain. This method is described in more detail in U.S. Pat. No. 6,165,745.

In another embodiment, the glycosylation of the antibody is modified. For example, deglycosylated antibodies (i.e., antibodies lack glycosylation) can be prepared. Such glycosylation modifications can be achieved, for example, by altering one or more glycosylation sites within the antibody sequence. For example, one or more amino acid substitutions can be made to eliminate glycosylation sites in one or more variable region frameworks and thus the glycosylation at those sites. Such deglycosylation can increase the affinity of the antibody for the antigen. See, e.g., U.S. Pat. Nos. 5,714,350 and 6,350,861.

Furthermore, antibodies with altered glycosylation, such as low-fucosylated antibodies with reduced fucose residues, or antibodies with increased bisecting GlcNac structures, can be made. It has been demonstrated that altered glycosylation increases the ADCC activity of the antibody. Such glycosylation modifications can be achieved, for example, by expressing the antibody in a host cell in which the glycosylation system is altered, which is known in the art and can be used as a host cell for expressing the recombinant antibodies disclosed herein to produce antibodies with altered glycosylation. For example, because cell lines Ms704, Ms705 and Ms709 lack the fucosyltransferase gene FUT8 (α(1,6)-fucosyltransferase), antibodies expressed in the Ms704, Ms705 and Ms709 cell lines lack fucose. The Ms704, Ms705 and Ms709 FUT8-/- cell lines were prepared by targeted disruption of the FUT8 gene in CHO/DG44 cells using two alternative vectors (see U.S. Pat. No. 20040110704 and Ohnuki et al., Biotechnol Bioeng. 87:614-22 (2004)). As another example, EP1,176,195 describes a cell line with disrupted FUT8 gene function. The gene encodes fucosyltransferase, such that antibodies expressed in such a cell line exhibit low fucosylation by reducing or eliminating α-1,6 bond-related enzymes. EP1,176,195 also describes a cell line with low or absent enzymatic activity for adding fucose to *N*-acetylglucosamine bound to the Fc region of an antibody, e.g., the rat myeloma cell line YB2/0 (ATCC CRL 1662). WO03/035835 describes Lee13 cells, a CHO variant cell line, which has a reduced ability to add fucose to Asn(297)-related sugars, which results in low fucosylation in the antibodies expressed by the host cell (see Shields et al., J. Biol. Chem. 277:26733-26740 (2002)). Antibodies with altered glycosylation characteristics can also be prepared in eggs, as described in WO06/089231. Alternatively, antibodies with altered glycosylation characteristics can be prepared in plant cells such as Lemna. WO99/54342 discloses a cell line that is genetically engineered to express glycosyltransferase that modifies glycoproteins (e.g., β(1,4)-*N*-acetylglucosamine transferase III (GnTIII)) such that antibodies expressed in the cell line exhibit increased bisecting GlcNac structures and thus have enhanced ADCC activity (see also Umana et al., Nat. Biotech. 17:176-180 (1999)). Alternatively, fucosidase is used to cut off the fucose residues of the antibody; for example, α-L-fucosidase removes the fucose residues from the antibody (Tarentino et al., Biochem. 14:5516-23 (1975)).

Another type of modification for the antibody disclosed herein is PEGylation. PEGylation of an antibody can, for example, increase the biological (e.g., serum) half-life of the antibody. To obtain a PEGylated antibody, an antibody or a fragment thereof is reacted with polyethylene glycol (PEG), e.g., a reactive ester or aldehyde derivative of PEG, under such conditions that one or more PEG groups are attached to the antibody or the antibody fragment. Preferably, the PEGylation is performed by acylation or alkylation with a reactive PEG molecule (or a similar reactive water-soluble polymer). The term "polyethylene glycol" as used herein includes any form of PEG for producing other protein derivatives, such as mono(C1-C10)alkoxy- or aryloxy-polyethylene glycol or polyethylene glycol-maleimide. In certain embodiments, the antibody to be PEGylated is a deglycosylated antibody. Methods of PEGylation are known in the art and can be applied to the antibody disclosed herein. See, e.g., EP0154316 and EP0401384.

### Physical properties of the antibody

The antibodies disclosed herein can be characterized by their various physical properties, and thus the categories they belong to are determined and/or distinguished. For example, an antibody may comprise one or more glycosylation sites in the light chain or heavy chain variable region. These glycosylation sites may result in increased immunogenicity of the antibody, or a change in the pK value of the antibody caused by altered antigen binding (Marshall et al., Annu Rev Biochem. 41:673-702 (1972); Gala and Morrison. J Immunol. 172:5489-94 (2004); Wallick et al.,J Exp Med. 168:1099-109 (1988); Spiro, Glycobiology. 12:43R-56R (2002); Parekh et al., Nature 316:452-7 (1985); and Mimura et al., Mol Immunol 37:697-706 (2000)). Glycosylation is known to occur in motifs containing N-X-S/T sequences. In certain cases, it is preferred that the ST2 antibody does not comprise variable region glycosylation. This can be achieved by selecting antibodies that do not contain glycosylation motifs in the variable regions or by mutating residues within the glycosylation region.

In one preferred embodiment, the antibody does not comprise an asparagine isomerization site. Deamidation of asparagine may occur in the N-G or D-G sequences and result in the production of isoaspartic acid residues, reducing stability.

Each antibody has a unique isoelectric point (pI), typically within the pH range of 6-9.5. The pI of IgG1 antibodies is typically within the pH range of 7-9.5, while that of IgG4 antibodies is typically within the pH range of 6-8. It is speculated that antibodies with pI values outside the normal range may undergo some unfolding and be unstable under *in vivo* conditions. Therefore, ST2 antibodies having a pI value within the normal range are preferred. This can be achieved by selecting antibodies with the pI within the normal range or by mutating surface residues.

### Nucleic acid molecule encoding the antibody disclosed herein

In another aspect, the present disclosure provides a nucleic acid molecule encoding the heavy chain and/or light chain variable regions or CDRs of the antibody disclosed herein. The nucleic acid may be present in intact cells, in cell lysates, or in partially purified or substantially pure form. The nucleic acid is "isolated" or "substantially pure" when purified from other cellular components or other contaminants, e.g., from other cellular nucleic acids or proteins, by using standard techniques. The nucleic acid disclosed herein may be, for example, DNA or RNA, and may or may not comprise intron sequences. In one preferred embodiment, the nucleic acid is a cDNA molecule.

The nucleic acid disclosed herein can be obtained using standard molecular biology techniques. For antibodies expressed by hybridomas (e.g., hybridomas prepared from transgenic mice carrying human immunoglobulin genes), light chain and heavy chain cDNAs encoding the antibodies prepared from the hybridomas can be obtained by standard PCR amplification or using cDNA cloning techniques. For antibodies obtained from immunoglobulin gene libraries (e.g., using phage display technology), nucleic acids encoding such antibodies can be recovered from the gene libraries.

Preferably, the nucleic acid molecule disclosed herein includes those encoding the VH and VL sequences or CDRs of the ST2 monoclonal antibody disclosed herein. Once the DNA fragments encoding the VH and VL fragments are obtained, operations such as converting the variable region genes into full-length antibody chain genes, Fab fragment genes or scFv genes can be further conducted using standard recombinant DNA techniques. In these operations, the DNA fragment encoding the VL or VH is operably linked to another DNA fragment encoding another protein, e.g., an antibody constant region or a flexible linker. The term "operably linked" as used herein means that two DNA fragments are joined together such that the amino acid sequences encoded by the two DNA fragments are both within the reading frame.

Isolated DNA encoding the VH region can be converted into a full-length heavy chain gene by operably linking the DNA encoding the VH to another DNA molecule encoding the heavy chain constant region (CH1, CH2 and CH3). The sequences of the human heavy chain constant region genes are known in the art, and these DNA fragments can be obtained by standard PCR amplification. The heavy chain constant region may be an IgG1, IgG2, IgG3, IgG4, IgA, IgE, IgM or IgD constant region, but is most preferably an IgG2 constant region. For the Fab fragment heavy chain gene, the DNA encoding the VH can be operably linked to another DNA molecule encoding only the heavy chain CH1 constant region.

Isolated DNA encoding the VL region can be converted into a full-length light chain gene (as well as an Fab light chain gene) by operably linking the DNA encoding the VL to another DNA molecule encoding the light chain constant region (CL). The sequences of the human light chain constant region genes are known in the art, and these DNA fragments can be obtained by standard PCR amplification. In preferred embodiments, the light chain constant region may be a κ or λ light chain constant region.

To prepare an scFv gene, a DNA fragment encoding the VH and VL is operably linked to another fragment encoding a flexible linker, for example, another fragment encoding the amino acid sequence (Gly₄-Ser)₃, such that the VH and VL sequences can be expressed as a continuous single-chain protein in which the VL and VH regions are linked by the flexible linker (see, e.g., Bird et al., Science 242:423-426 (1988); Huston et al., Proc Nat. Acad. Sci. USA 85:5879-5883 (1988); and McCafferty et al., Nature 348:552-554 (1990)).

### Preparation of the monoclonal antibody disclosed herein

The monoclonal antibody (mAb) disclosed herein can be prepared using the somatic hybridization (hybridoma) technique in Kohler and Milstein Nature. 256:495 (1975). Other embodiments for preparing the monoclonal antibody include viral or oncogenic transformation of B lymphocytes and phage display techniques. Chimeric or humanized antibodies are also well known in the art. See, e.g., U.S. Pat. Nos. 4,816,567; 5,225,539; 5,530,101; 5,585,089; 5,693,762 and 6,180,370.

### Transfectoma for preparing the monoclonal antibody

The antibody disclosed herein can also be produced in host cell transfectomas using, for example, recombinant DNA techniques in combination with gene transfection methods (e.g., Morrison, S. Science 229:12021985). In one embodiment, DNA encoding partial or full-length light and heavy chains obtained using standard molecular biology techniques is inserted into one or more expression vectors such that the gene is operably linked to transcriptional and translational regulatory sequences. In this case, the term "operably linked" refers to linking the antibody gene into the vector such that the transcriptional and translational regulatory sequences in the vectors perform their intended functions of regulating the transcription and translation of the antibody gene.

The term "regulatory sequence" includes promoters, enhancers and other expression control elements (e.g., polyadenylation signals) that control the transcription or translation of the antibody gene. Such regulatory sequences are described in Goeddel (Gene Expression Technology. Methods in Enzymology 185, Academic Press, San Diego, CA (1990)). Preferably, regulatory sequences for expression in a mammalian host cell include viral elements that direct the high-level protein expression in mammalian cells, such as promoters and/or enhancers derived from cytomegalovirus (CMV), simian virus 40 (SV40), adenoviruses, e.g., the adenovirus major late promoter (AdMLP). Alternatively, non-viral regulatory sequences such as ubiquitin promoters or β-globin promoters are used. In addition, the regulatory elements consist of sequences of different origins, such as the SRα promoter system, which comprises the sequence from the SV40 early promoter and the sequence of the long terminal repeat of the human T-cell leukemia type I virus (Takebe et al., Mol Cell. Biol. 8:466-472 (1988)). The expression vector and expression regulatory sequences are compatible with the expression host cell used.

The antibody light chain gene and the antibody heavy chain gene can be inserted into the same expression vector or different expression vectors. In preferred embodiments, variable regions are inserted into an expression vector that has encoded the heavy chain constant region and the light chain constant region of the desired subtype to construct a full-length antibody gene, such that the VH is operably linked to the CH in the vector and the VL is operably linked to the CL in the vector. Alternatively, the recombinant expression vector can encode a signal peptide that facilitates the secretion of antibody chains from the host cell. The antibody chain gene can be cloned into a vector such that the signal peptide is linked to the amino terminus of the antibody chain gene in the reading frame. The signal peptide may be an immunoglobulin signal peptide or a heterologous signal peptide (i.e., a signal peptide from a non-immunoglobulin protein).

In addition to the antibody chain genes and regulatory sequences, the recombinant expression vector disclosed herein may carry other sequences, such as a sequence that regulates replication of the vector in the host cell (e.g., an origin of replication) and a selectable marker gene. The selectable marker gene can be used to select host cells into which the vector has been introduced (see, e.g., U.S. Pat. Nos. 4,399,216; 4,634,665 and 5,179,017). For example, the selectable marker gene typically imparts resistance to a drug such as G418, hygromycin or methotrexate to the host cell into which the vector has been introduced. Preferred selectable marker genes include the dihydrofolate reductase (DHFR) gene (for methotrexate selection/amplification in DHFR host cells) and the neo gene (for G418 selection).

To express the light chain and the heavy chain, host cells are transfected with expression vectors encoding the heavy chain and the light chain using standard techniques. The term "transfect" encompasses a variety of techniques for introducing exogenous DNA into prokaryotic or eukaryotic host cells, such as electroporation, calcium phosphate precipitation, and DEAE-dextran transfection. Although expressing the antibody disclosed herein in prokaryotic or eukaryotic host cells is theoretically feasible, expressing the antibody in eukaryotic cells is preferred, and expressing the antibody in mammalian host cells is most preferred. This is because eukaryotic cells, particularly mammalian cells, are more likely to assemble and secrete properly folded and immunologically active antibodies than prokaryotic cells.

Preferred mammalian host cells for expressing the recombinant antibody disclosed herein include Chinese hamster ovary cells (CHO cells) (including dhfr-CHO cells administered with a DHFR selectable marker, as described in Urlaub and Chasin, Proc. Natl. Acad. Sci. USA 77:4216-4220 (1980); the DHFR selectable marker is as described in RJ Kaufman and PA Sharp. J. Mol. Biol. 159:601-621 (1982)), NSO myeloma cells, COS cells and SP2 cells. Another preferred expression system, particularly when NSO myeloma cells are used, is the GS gene expression system disclosed in WO87/04462, WO89/01036 and EP338,841. When a recombinant expression vector encoding an antibody gene is introduced into a mammalian host cell, the antibody is prepared by culturing the host cell for a period of time sufficient to allow the expression of the antibody in the host cell, or preferably, sufficient to allow the secretion of the antibody into the medium in which the host cell grows. The antibody can be recovered from the culture medium using protein purification methods.

### Polypeptide fusion

In another aspect, the present disclosure relates a polypeptide fusion comprising one or more of the antibodies or the antigen-binding fragments thereof disclosed herein, wherein the antibodies or the antigen-binding fragments thereof disclosed herein are linked to at least one other functional molecule, which may be a peptide or a protein or a non-protein. In one embodiment, the polypeptide fusion disclosed herein includes immunoconjugates comprising one or more of the antibodies or the antigen-binding fragments thereof disclosed herein and at least one therapeutic agent, such as a steroid, linked to the antibodies or the antigen-binding fragments thereof disclosed herein. In one embodiment, the polypeptide fusion disclosed herein includes bifunctional molecules comprising one or more of the antibodies or the antigen-binding fragments thereof disclosed herein and at least one immune cytokine or receptor ligand linked to the antibody or the antigen-binding fragment thereof disclosed herein, wherein the immune cytokine or receptor ligand may be for IgE, IL-4, IL-4R, IL-5, IL-5R, IL-6, IL-9, IL13, IL13R, IL-17, IL-23, IL-33, OX40 ligand (OX40L), GM-CSF or TSLP, or TSLPR/IL7R. In one embodiment, the bifunctional molecules have a third function in addition to the Fc receptor binding function and the ST2 binding function. The third function may be for IgE, IL-4, IL-4R, IL-5, IL-5R, IL-6, IL-9, IL13, IL13R, IL-17, IL-23, IL-33, OX40 ligand (OX40L), GM-CSF or TSLP, or TSLPR/IL7R. As used herein, "bifunctional molecule" encompasses molecules having three or more functions. In other embodiments, the polypeptide fusion disclosed herein also includes other forms. These and the other forms of the polypeptide fusion can be prepared by genetic engineering, by using chemical methods, etc.

### Multispecific molecule

In another aspect, the present disclosure relates to a multispecific molecule comprising one or more of the antibodies or the antigen-binding fragments thereof disclosed herein, wherein the antibodies or the antigen-binding fragments thereof disclosed herein are linked to at least one other functional portion with specificity that is different from that of the antibodies or the antigen-binding fragments disclosed herein, the other functional portion comprising another peptide or protein (e.g., another antibody or an antigen-binding fragment thereof) to produce a multispecific molecule that binds to at least two different binding sites or targets. Therefore, "multispecific molecule" as used herein encompasses molecules with two types of specificity (i.e., bispecific molecules), three types of specificity (i.e., trispecific molecules), four types of specificity (i.e., tetraspecific molecules), or more specificities.

In one specific embodiment, the multispecific molecule disclosed herein may have one or more other types of specificity in addition to the anti-Fc binding specificity and the ST2 binding specificity. Illustratively, the other types of specificity may be for IgE, IL4, IL4R, IL5, IL5R, IL6, IL9, IL13, IL13R, IL17, IL23, IL33 or TSLP, or TSLPR/IL7R.

In one specific embodiment, the multispecific molecule may be present in a variety of different forms and sizes. Illustratively, in terms of the bispecific molecule, at one end of the size spectrum, the bispecific molecule remains in conventional antibody form except that it has two binding arms with different types of specificity rather than two binding arms with the same specificity. At the other end, the bispecific molecule consists of two single-chain antibody fragments (scFv) linked by a peptide chain, known as the Bs(scFv)₂ construct. Medium-sized bispecific molecules comprise two different F(ab) fragments linked by a peptide linker. These and other forms of the bispecific molecule can be prepared by genetic engineering or somatic hybridization, or by using chemical methods. See, e.g., Kufer et al., cited supra; Cao and Suresh, Bioconjugate Chemistry. 9(6).635-644 (1998); and van Spriel et al., Immunology Today. 21(8):391-397 (2000).

### Viral vector

In another aspect, the antibody or the antigen-binding fragment thereof disclosed herein can also be encoded or carried by a viral vector. In addition, the antibody or the antigen-binding fragment thereof disclosed herein may also be used with the viral vector, or the viral vector encoding or carrying the antibody or the antigen-binding fragment thereof disclosed herein is introduced into humans.

The present disclosure also provides a pharmaceutical composition comprising the polypeptide fusion, the multispecific molecule and the viral vector disclosed herein and a pharmaceutically acceptable excipient, diluent or carrier.

### Pharmaceutical composition

In another aspect, the present disclosure provides a pharmaceutical composition comprising the antibody or the antigen-binding fragment thereof disclosed herein formulated together with a pharmaceutically acceptable excipient, diluent or carrier. The pharmaceutical composition may optionally comprise one or more other pharmaceutically active ingredients, such as another antibody or drug, e.g., another ST2 antibody, an anti-IgE antibody, another anti-inflammatory drug, an anti-asthma drug, an anti-chronic obstructive pulmonary disease drug, an anti-ulcerative colitis drug, an anti-atopic dermatitis drug or an anti-psoriasis drug.

Preferably, the pharmaceutical composition is suitable for intravenous, intramuscular, subcutaneous, parenteral, spinal or epidermal administration (e.g., by injection or infusion). Depending on the route of administration, the active ingredient may be encapsulated in a material to protect it from acids and other natural conditions that may inactivate it. "Parenteral administration" refers to a mode that is different from enteral administration and topical administration and that is typically performed by injection, including but not limited to, intravenous, intramuscular, intraarterial, intramembranous, intracapsular, intraorbital, intracardiac, intradermal, intraperitoneal, transtracheal, subcutaneous, subcuticular, intraarticular, subcapsular, subarachnoid, intraspinal, epidural and intrasternal injection and bolus injection. Alternatively, the pharmaceutical composition disclosed herein may be administered through a non-parenteral route, e.g., topical, epidermal or mucosal administration, e.g., intranasal, oral, vaginal, rectal, sublingual or topical administration.

The pharmaceutical compositions may be in the form of sterile aqueous solutions or dispersions. They may also be formulated in microemulsions, liposomes or other ordered structures suitable for high concentrations of drugs.

The administration regimen is adjusted to provide the best desired response (e.g., therapeutic response). For example, a single large dose may be administered, several divided doses may be administered over time, or the dose may be proportionally reduced or increased as indicated by the exigencies of the therapeutic situation. It is particularly advantageous to formulate parenteral compositions in dosage units for ease of administration and uniformity of dosage. Dosage units refers to physically separate units that are suitable for single administration to a treated subject; each unit contains a predetermined amount of the active ingredient calculated to produce the desired therapeutic effect in association with the pharmaceutical carrier. Alternatively, the antibody may be administered in a sustained-release formulation, in which case the frequency of administration required is reduced. For administration of the composition, the dose may be about 0.0001 to 100 mg/kg of host body weight, more usually 0.01 to 5 mg/kg. For example, the dose may be 0.3 mg/kg of body weight, 1 mg/kg of body weight, 3 mg/kg of body weight, 5 mg/kg of body weight or 10 mg/kg of body weight or within the range of 1-10 mg/kg of body weight. Exemplary treatment regimens require administration twice every week, once every week, once every two weeks, once every three weeks, once every four weeks, once every month, once every two months, once every three months, or once every three to six months.

The pharmaceutical composition may be a sustained-release agent, including implants, transdermal patches, and microencapsulated delivery systems. Biodegradable and biocompatible polymers such as ethylene-vinyl acetate, polyanhydrides, polyglycolic acid, collagen, polyorthoesters and polylactic acid may be used.

In certain embodiments, the antibody disclosed herein may be formulated to ensure proper *in vivo* distribution. For example, to ensure that the therapeutic antibody disclosed herein crosses the blood-brain barrier, the antibody may be formulated in liposomes, which may additionally contain targeting functional groups to enhance selective delivery to particular cells or organs.

### Use and method of the present disclosure

The antibody or the antigen-binding fragment thereof (the encoding nucleic acid molecule, the pharmaceutical composition, the polypeptide fusion, the multispecific molecule, or the viral vector) disclosed herein has a variety of *in vitro* and *in vivo* applications relating to the diagnosis, treatment and/or prevention of IL33/ST2-mediated related diseases and conditions. The IL33/ST2-mediated related diseases and conditions include, but are not limited to, asthma, allergic rhinitis, chronic obstructive pulmonary disease, eosinophilic bronchitis, eosinophilic esophagitis, atopic dermatitis, psoriasis, systemic lupus erythematosus, pemphigoid, rheumatoid arthritis, ankylosing spondylitis, inflammatory bowel disease, pulmonary fibrosis, hepatic fibrosis, systemic sclerosis, sarcoidosis, graft-versus-host disease (GVHD), diabetes, cardiovascular diseases, or combinations thereof. The antibody or the antigen-binding fragment thereof disclosed herein can be administered to human subjects to alleviate, ameliorate or treat the diseases or conditions. In a diagnostic process, the antibody disclosed herein can be contacted with a sample of a subject under conditions that allow the antibody or the antigen-binding fragment thereof disclosed herein to bind to ST2 to detect the amount of the ST2 protein in the sample of the subject.

These and other methods of the present disclosure are discussed further below.

### Combination therapy

The present disclosure provides co-administration of the ST2 antibody or the antigen-binding fragment thereof (the encoding nucleic acid molecule, the pharmaceutical composition, the polypeptide fusion, the multispecific molecule, or the viral vector) disclosed herein with one or more other antibodies or drugs that can alleviate, ameliorate or treat IL33/ST2-mediated related diseases and conditions in a subject, the IL33/ST2-mediated related diseases and conditions including but not limited to asthma, allergic rhinitis, chronic obstructive pulmonary disease, eosinophilic bronchitis, eosinophilic esophagitis, atopic dermatitis, psoriasis, systemic lupus erythematosus, pemphigoid, rheumatoid arthritis, ankylosing spondylitis, inflammatory bowel disease, pulmonary fibrosis, hepatic fibrosis, systemic sclerosis, sarcoidosis, graft-versus-host disease (GVHD), diabetes, cardiovascular diseases, or combinations thereof. In one embodiment, the present disclosure provides a method for treating asthma, chronic obstructive pulmonary disease or atopic dermatitis in a subject, wherein the ST2 antibody or the antigen-binding fragment thereof disclosed herein is administered together with one or more other antibodies, e.g., a TSLP antibody, a TSLPR antibody, an IL4 antibody, an IL4R antibody, an IL13 antibody, an IL13R antibody, an IL5 antibody, an IL5R antibody, and/or an IgE antibody. In another embodiment, the present disclosure provides a method for treating asthma, chronic obstructive pulmonary disease or atopic dermatitis in a subject, wherein the ST2 antibody or the antigen-binding fragment thereof disclosed herein is administered together with at least one additional drug, e.g., an anti-asthma drug, an anti-chronic obstructive pulmonary disease drug, or an anti-atopic dermatitis drug. In certain embodiments, the subject is a human. Other therapies that can be used in combination with the ST2 antibody or the antigen-binding fragment thereof disclosed herein also include: reducing or avoiding allergen exposure, hormone therapy, surgery, etc.

The combination of therapeutic agents described herein can be administered simultaneously as a single composition in a pharmaceutically acceptable carrier, or administered simultaneously as separate compositions, wherein each agent is in a pharmaceutically acceptable carrier. In another embodiment, the combination of therapeutic agents may be administered sequentially.

Furthermore, if the combination therapy is administered multiple times and the agents are administered sequentially, the sequence in the sequential administration at each time point can be reversed or maintained, and the sequential administration can be combined with simultaneous administration or any combination thereof.

Although the foregoing invention has been described in detail by providing illustrations and examples for the purpose of clear understanding, it will be apparent to those of ordinary skill in the art in light of the teachings of the present disclosure that certain changes and modifications can be made to the present disclosure without departing from the spirit or scope of the appended claims. The present disclosure is further explained by the description of the following examples, which are not intended to be limiting. Those skilled in the art will readily identify a variety of noncritical parameters that may be changed or modified to produce substantially similar results.

Unless otherwise indicated, the practice of the present disclosure will use conventional methods in protein chemistry, biochemistry, the recombinant DNA technique, and pharmacology within the art.

### Examples

### Example 1. Preparation of ST2 Antigen and Test Protein

A full-length gene of human UniProt Interleukin-1 receptor-like 1 (ST2) isoform A (SEQ ID NO: 1) (hST2) was used as a template of the ST2 of the present disclosure to obtain a gene sequence that encoded the antigen and the protein for detection of the present disclosure, which could be recombined with a mouse antibody heavy chain Fc fragment (such as mouse IgG2a) to form hST2-mFc or recombined with a human antibody heavy chain Fc fragment to form hST2-hFc for immunization of mice or later screening assays. cDNAs that encoded an recombinant protein containing hST2 extracellular domain with mouse antibody heavy chain Fc tag (hST2-ECD-mFc, SEQ ID NO: 2) and an recombinant protein containing hST2 extracellular domain with human antibody heavy chain Fc tag (hST2-ECD-hFc, SEQ ID NO: 4) (SEQ ID NOs: 3 and 5, respectively) were obtained by gene synthesis and were respectively subcloned into pcDNA3.1 expression vectors (Invitrogen, V-790), and Expi293 cells (Thermo, A14527) were transfected with the vectors constructed above for transient expression. The hST2-ECD-mFc and hST2-ECD-hFc recombinant proteins were then purified through a Protein A column (GE healthcare).

cDNA that encoded a recombinant protein containing human IL33 with Avitag (SEQ ID NO: 7) was obtained by gene synthesis and was cloned into a GST-tagged expression vector, and the vector constructed above was transformed into BL21 competent cells for induced expression. The GST-IL33 Avitag protein was purified through a GST purification column and digested with Thrombin enzyme (Sigma, T4648-1KU), and then GST was removed to obtain hIL33 Avitag (SEQ ID NO: 6).

cDNA that encoded a human IL33 recombinant protein (SEQ ID NO: 9) was obtained by gene synthesis and was cloned into a GST-tagged expression vector, and the vector constructed above was transformed into BL21 competent cells for induced expression. The GST-IL33 protein was purified through a GST purification column and digested with Thrombin enzyme (Sigma, T4648-1KU), and then GST was removed to obtain hIL33 protein (SEQ ID NO: 8).

The ST2 antibody RG6149 in the examples refers to the antibody Ab2 in CN104334582, which was prepared in-house, and the amino acid sequences of the heavy chain and light chain of which are set forth in SEQ ID NO: 83 and SEQ ID NO: 84 of the present disclosure. The ST2 antibody GSK3772847 refers to the antibody STLM208 in CN104411333, which was prepared in-house, and the the amino acid sequences of heavy chain and light chain of which are set forth in SEQ ID NO: 85 and SEQ ID NO: 86 of the present disclosure.

### Example 2. Preparation of Anti-ST2 Hybridoma Monoclonal Antibodies

The purified hST2-ECD-mFc recombinant protein (100 µg/mouse) was mixed well with an equal volume of complete Freund's adjuvant (Sigma, F5881-10X10ML) (prime immunization) or incomplete Freund's adjuvant (Sigma, F5506-10X10ML) (boost immunization) and emulsified, and BALB/c mice were immunized subcutaneously with the resulting emulsion every 2 weeks over 8 weeks. Three days before fusion, the mice were immunized with adjuvant-free hST2-ECD-mFc antigen (50 µg/mouse) as a booster by intraperitoneal injection. The spleen cells from the immunized mice (1 × 10⁸) were fused with SP2/0 myeloma cells (2 × 10⁷) by following a PEG-mediated fusion procedure to give hybridoma cells. After fusion, the cells were resuspended in HAT complete medium (Gibco, 21060017), distributed into a 96-well plate at 0.1 mL/well, and cultured in a 37 °C, 5% COz incubator. In general, about 10-15 days after fusion, the cell culture supernatants were tested for the binding activity for ST2 by ELISA (see Example 5), and the cell culture supernatants in the wells that tested positive were selected and tested for the blocking activity against IL33/ST2 binding by ELISA (see Example 6).

The wells in which ST2 could be specifically bound to and IL33/ST2 binding could be blocked were selected, and the contents in the wells were expanded into a 24-well plates in time depending on the cell density. After the cell strains transferred into the 24-well plate were retested, conservation and first subcloning were performed. The cell strains tested positive after the first subcloning were selected for conservation, and second subcloning was performed. The cell strains tested positive after the second subcloning were subjected to conservation and protein expression.

### Example 3. Acquisition of cDNA of anti-ST2 Antibody and Construction of Chimeric Antibodies

The total RNA was isolated as a template from the above hybridoma cells with binding and blocking functions using a total RNA extraction kit, and a first strand of cDNA was synthesized using superscript III reverse transcriptase (Thermo, 18080051) according to the instructions. The variable region sequences of the antibodies were then amplified by PCR reactions using degenerate mouse IgG primers.

The PCR mixture was electrophoretically separated in a 1% agarose/Tris-borate gel containing 0.5 µg/mL ethidium bromide. A DNA fragment of the expected size (about 500 bp for heavy and light chains) was excised from the gel and purified. The purified PCR product was cloned into pMD-19T vector (Takara, 6013) and transformed into DH5α competent *Escherichia coli* (Takara, 9057). Five colonies were picked from an LB solid culture plate and subjected to DNA sequencing. The heavy chain variable region sequences and the light chain variable region sequences of the antibodies were obtained: 7D1 (SEQ ID NOs: 35, 36), 3C6 (SEQ ID NOs: 37, 38), 31C8 (SEQ ID NOs: 39, 40), 26A1 (SEQ ID NOs: 41, 42), 8F4 (SEQ ID NOs: 43, 44), and 17E2 (SEQ ID NOs: 45, 46).

Construction and expression of chimeric antibodies: The mouse VL region gene synthetic fragment was ligated to the human κ chain constant region by double digestion to construct a chimeric light chain. The mouse VH region gene synthetic fragment was ligated to the human IgG2 constant region by double digestion to construct a chimeric heavy chain.

Expi CHO cells (a 50 mL system, 6 × 10⁶/mL cells, 1 µg/mL DNA) were co-transfected with DNA vector containing the chimeric light chain described above and DNA vector containing the chimeric heavy chain described above for transient expression and cultured for 7 days. The chimeric antibodies in the cell culture supernatant was then purified through a Protein A column (GE healthcare).

### Example 4. Determination of Affinity of Anti-ST2 Antibodies for hST2 Antigen

An anti-mouse IgG antibody (for capturing human ST2 antigen, Cytiva, 29215281) or an anti-his antibody (for capturing cynomolgus monkey ST2 antigen, Cytiva, 29234602) were coupled onto CM5 biochip (Cytiva, BR-1000-12) using the method described in the product's instructions, and an anti-ST2 antibody with a series of concentrations (32.8 nM, 16.4 nM, 8.2 nM, 4.1 nM, 2.05 nM, 1.0259 nM, and 0.51297 nM) was allowed to flow over the chip surface. The reaction signals were detected in real time using a Biacore instrument (Cytiva, BiacoreT200), and then an association-dissociation curve was obtained. After the completion of dissociation in each cycle, the biochip was regenerated with a regeneration solution for the next capture; the procedure was repeated until the affinity of the different antibodies for ST2 was determined. The acquired data were finally analyzed using the GE BIAevaluation software with a 1:1 (Langmuir) binding model to determine the association rate constants ka (kon) and the dissociation rate constants kd (koff), and the dissociation constants KD were given by KD = kd/ka. The determined affinity of the anti-ST2 antibody for human ST2 antigen (hST2-ECD-mFc), cynomolgus monkey ST2 antigen (Cyno-ST2-his, Sino biological, 90915-C08H) and the determined affinity of the anti-ST2 chimeric antibodies for ST2 were shown in Table 5.

**Table 5. The affinity of the anti-ST2 chimeric antibodies for ST2**

| Antibody | KD(M) | |
|---|---|---|
| | hST2 | Cyno-ST2 |
| RG6149 | 6.86E-11 | 4.90E-11 |
| xi7D1 | 1.45E-10 | 1.24E-10 |
| xi3C6 | 8.72E-11 | 1.52E-07 |
| xi31C8 | 1.77E-11 | 3.23E-10 |
| xi26A1 | 2.99E-11 | 6.98E-08 |
| xi8F4 | 9.33E-11 | 4.04E-09 |
| xi17E2 | 5.41E-11 | 2.57E-11 |

### Example 5. Binding Assays of Anti-ST2 Antibodies Based on ELISA

ST2 binding screening and analysis of antibodies were performed by ELISA using hST2-ECD-mFc (for assays of chimeric and humanized antibodies), hST2-ECD-hFc protein (for assays of hybridoma antibodies) and Cyno-ST2-his (Sino biological, 90915-C08H) as antigens. A high-adsorption 96-well plate (Costar, 9018) was coated with 2 µg/mL hST2-ECD-mFc or Cyno-ST2-his antigen at 100 µL/well and incubated overnight at 4 °C. After the antigen not adsorbed was well washed off, the non-specific binding sites were blocked with a blocking buffer (PBS containing 2% bovine serum albumin). After the plate was washed three times with a washing buffer (PBS containing 0.05% (v/v) Tween 20), the anti-ST2 antibodies (serially diluted 3-fold from an initial concentration of 10 nM to 8 concentrations: 3.333 nM, 1.111 nM, 0.370 nM, 0.123 nM, 0.041 nM, 0.014 nM, 0.005 nM, and 0.002 nM) were added at 100 µL/well, and the plate was incubated at room temperature for 1 h. After the plate was washed with the washing buffer, a horseradish peroxidase (HRP)-conjugated secondary antibody was added, and the plate was incubated for another 60 min. After the plate was washed with the washing buffer, the substrate TMB solution (Thermo, 00-4201-56) was added at 100 µL/well, and the plate was incubated at room temperature for 2 min. A stop solution (2 N H₂SO₄) was added at 100 µL/well to terminate the reactions. Colorimetric signals were generated and read at 450 nm using a microplate reader (PE, Envision). The data were analyzed using GraphPad Prism5, and the EC₅₀ values were calculated. The EC₅₀ values for the binding of the anti-ST2 chimeric antibodies to hST2 and Cyno-ST2 are shown in Table 6 and FIGs. 1-2.

**Table 6. The EC₅₀ values for the binding of the anti-ST2 chimeric antibodies to hST2 and Cyno-ST2 in the ELISA assays**

| Antibody | EC50(nM) | |
|---|---|---|
| | hST2 | Cyno-ST2 |
| RG6149 | 0.113 | 0.129 |
| xi7D1 | 0.191 | 0.191 |
| xi3C6 | 0.157 | 0.430 |
| xi31C8 | 0.184 | 0.163 |
| xi26A1 | 0.175 | 0.946 |
| xi8F4 | 0.172 | 0.164 |
| xi17E2 | 0.235 | 0.220 |

### Example 6. Blocking Assays of Anti-ST2 Antibodies based on ELISA

Whether the anti-ST2 antibodies were able to block the binding of hIL33 to hST2 was determined based on ELISA. A high-adsorption 96-well plate was coated with 2 µg/mL hST2-ECD-hFc at 100 µL/well and incubated overnight at 4 °C. After the antigen not adsorbed was well washed off, the non-specific binding sites were blocked with a blocking buffer (PBST containing 1% bovine serum albumin). After the plate was washed three times with a washing buffer (PBST, PBS containing 0.05% (v/v) Tween 20), the anti-ST2 antibodies (serially diluted 2-fold from an initial concentration of 66.67 nM to 8 concentrations) were added at 100 µL/well, and the plate was incubated at 37 °C for 1 h. After the plate was washed 3 times with the washing buffer, 100 µL of biotin-labeled hIL33 Avitag (with a concentration of 0.1 µg/mL) was added to each well, and the plate was incubated at 37 °C for 1 h. After the plate was washed 3 times with the washing buffer, a 1:1000 diluted secondary antibody Avidin HRP (Jackson immunoresearch, 016-030-084) was added at 100 µL/well, and the plate was incubated at room temperature for 1 h. After the plate was washed with the washing buffer, the substrate TMB solution (Thermo, 00-4201-56) was added at 100 µL/well, and the plate was incubated at room temperature for 3 min. 50 µL of a stop solution (2 N H₂SO₄) was added to terminate the reactions. Colorimetric signals were generated and read at 450 nm using a plate reader (PE, Envision). The data were analyzed using GraphPad Prism5, and the IC₅₀ values were calculated. The IC₅₀ values for the anti-ST2 chimeric antibodies' blocking of IL33/ST2 binding are shown in Table 7 and FIG. 3.

**Table 7. The IC₅₀ values for the anti-ST2 chimeric antibodies' blocking of IL33/ST2 binding in the ELISA assays**

| Antibody | IC50(nM) |
|---|---|
| RG6149 | 2.882 |
| xi7D1 | 2.895 |
| xi3C6 | 2.518 |
| xi31C8 | 2.498 |
| xi26A1 | 2.529 |
| xi8F4 | 2.787 |
| xi17E2 | 3.346 |

### Example 7. Cell-Based Binding Assays of Anti-ST2 Antibodies

The binding capacity of the anti-ST2 antibodies to hST2-overexpressing cell lines and cyno-ST2-overexpressing cell lines was analyzed using an FACS-based method through binding assays with the hST2 (SEQ ID NO:1)-overexpressing HEK293T cell line (HEK293T-hST2-NFκB-Luciferase) and the cynomolgus monkey ST2 (SEQ ID NO: 10)-overexpressing HEK293T cell line (HEK293T-Cyno-ST2-NFxB-Luciferase). HEK293 cells were transfected with the pLenti6.3- hST2 plasmid and the pLenti6.3-NFxB-luciferasae plasmid using a lentiviral transfection system to construct the cell line HEK293T-hST2-NFxB-Luciferase. HEK293T cells were transfected with the pLenti6.3-cynoST2 plasmid and the pLenti6.3-NFxB-luciferasae plasmid to construct the cell line HEK293T-Cyno-ST2-NFκB-Luciferase. 3 × 10⁵ HEK293T-hST2-NFxB-Luciferase or HEK293T-Cyno-ST2-NFxB-Luciferase cells were added to a 96-well culture plate. Serially diluted anti-ST2 antibodies (serially diluted 4-fold from an initial concentration of 667 nM to 8 concentrations in binding assays with the HEK293T-hST2-NFxB-Luciferase cells; serially diluted 4-fold from an initial concentration of 400 nM to 8 concentrations in binding assays with the HEK293T-Cyno-ST2-NFxB-Luciferase cells) were added to the cell suspensions. After the plate was incubated at room temperature for 60 min, the cells were washed 3 times with PBS. A 1:200 diluted PE goat-anti-human-IgG secondary antibody (Jackson immunoresearch, 109-116-170) was added at 100 µL/well, and the plate was incubated at room temperature for 30 min. The cells were washed 3 times with PBS and resuspended in 100 µlL of PBS, and then the fluorescence signals were analyzed and detected using a flow cytometer (BD, Accuri C6). The binding capacity of the anti-ST2 antibodies to hST2 and cyno-ST2 on the cell line surfaces was measured in terms of the mean fluorescence intensity (MFI) of staining. The data were analyzed using GraphPad Prism5, and the EC₅₀ values were calculated. The EC₅₀ values for the binding of the anti-ST2 chimeric antibodies to hST2 and Cyno-ST2 at the cellular level are shown in Table 8 and FIGs. 7-8.

**Table 8. The EC₅₀ values for the binding of the anti-ST2 chimeric antibodies to hST2 and Cyno-ST2 at the cellular level**

| Antibody | EC50(nM) | |
|---|---|---|
| | HEK293T-hST2-NFκB-Luciferase | HEK293T-Cyno-ST2-NFκB-Luciferase |
| RG6149 | 0.589 | 0.647 |
| xi7D1 | 0.265 | 0.394 |
| xi3C6 | 0.349 | 104.6 |
| xi31C8 | 0.298 | 0.830 |
| xi26A1 | NA | 415.3 |
| xi8F4 | 0.343 | 0.458 |
| xi17E2 | 0.224 | 0.487 |

| | | |
|---|---|---|
| NA: limit of detection not reached. | | |

### Example 8. Assays of Anti-ST2 Antibodies Blocking Interaction Between IL33 Protein and hST2-Overexpressing Cell Line (HEK293T-hST2-NFκB-Luciferase)

HEK293T cells naturally express IL1RAcP protein at a high level. On this basis, HEK293 cells were transfected with pLenti6.3-hST2 plasmid and pLenti6.3-NFxB-Luciferasae plasmid using a lentivirus transfection system to construct a stably transfected cell line HEK293T-hST2-NFxB-Luciferase. IL33 binds to ST2 and recruits IL-1RAcP. In this way the downstream NFxB signaling pathway can be activated and thus the expression of Luciferase is started. When an anti-ST2 antibody is present in the system, the binding of IL33 to ST2, and thus the expression of Luciferase, can be blocked. The blocking activity of the anti-ST2 antibodies was evaluated by detecting the changes in the intensity of fluorescence of the reaction substrate. HEK293T-hST2-NFxB-Luciferase cells at log phase in good conditions were taken and diluted to 2.5 × 10⁵ cells/mL. The cell dilution was added to a 384-well plate (Thermo, 262360) at 20 µL/well. Then serially diluted anti-ST2 antibodies (serially diluted 4-fold from an initial concentration of 667 nM) were added at 15 µL/well. The plate was incubated at 37 °C for 30 min. After the incubation, 15 µL of hIL33 protein was added to each well (with a final concentration of 50 pM). The mixtures were well mixed and incubated at 37 °C for 5 h. Then 50 µL of ONE-Glo^{™} Luciferase Assay (Promega, E6120) was added to each well, and the mixtures were reacted in the dark for 2-5 min. The chemiluminescent signals were read using a microplate reader (PE, Envision). The data were analyzed using GraphPad Prism5, and the IC₅₀ values were calculated. The IC₅₀ values for the anti-ST2 chimeric antibodies' blocking of the IL33/ST2 signaling at the cellular level are shown in Table 9 and FIG. 9.

**Table 9. The IC₅₀ values for the anti-ST2 chimeric antibodies' blocking of the IL33/ST2 signaling at the cellular level**

| Antibody | IC50(nM) |
|---|---|
| | HEK293T-hST2-NFκB-Luciferase |
| RG6149 | 11.880 |
| xi7D1 | 0.120 |
| xi3C6 | 0.673 |
| xi31C8 | 2.534 |
| xi26A1 | 7.567 |
| xi8F4 | ND |
| xi17E2 | ND |

| | |
|---|---|
| "ND" indicates "not detected". | |

### Example 9. Assays of Anti-ST2 Antibodies Blocking Interaction Between IL33 Protein and Cells Naturally Expressing hST2 (KU812-NFxB-Luciferasae)

KU812 cells naturally express ST2 and IL1RAcP protein. KU812 cells were transfected with pLenti6.3-NFxB-Luciferasae using a lentivirus transfection system to construct a stably transfected cell line KU812-NFκB-Luciferasae. The experimental principle according to Example 8. KU812-NFκB-Luciferasae cells at log phase in good conditions were taken and diluted to 2.5 × 10⁵ cells/mL. The cell dilution was added to a 384-well plate (Thermo, 262360) at 20 µL/well. Then serially diluted anti-ST2 antibodies (serially diluted 4-fold from an initial concentration of 667 nM) were added at 15 µL/well. The plate was incubated at 37 °C for 30 min. After the incubation, 15 µL of hIL33 protein was added to each well (with a final concentration of 200 pM). The mixtures were well mixed and incubated at 37 °C for 5 h. Then 50 µL of ONE-Glo^{™} Luciferase Assay (Promega, E6120) was added to each well, and the mixtures were reacted in the dark for 2-5 min. The chemiluminescent signals were read using a microplate reader (PE, Envision). The data were analyzed using GraphPad Prism5, and the IC₅₀ values were calculated.

### Example 10. Assays of Anti-ST2 Antibodies Blocking Co-Stimulatory Interaction Between IL33 and IL2 and CD4⁺ T Cells

Under the co-stimulation by IL33 and IL2, IL33 binds to ST2 on the CD4⁺ T cell surface, activating the downstream signaling pathway to induce the secretion of the cytokine IL5. When an anti-ST2 antibody is present in the system, it can block the binding of IL33 to ST2 on CD4⁺ T cells, inhibiting the IL5 secretion. The capacity of the anti-ST2 antibodies to block the co-stimulatory interaction between IL33 and IL2 and CD4⁺ T cells was analyzed by measuring the IL5 content.

CD4⁺ T cells were sorted from human PBMCs using human CD4⁺ T cell sorting kit (stemcell, 17952) and plated in a 96-well U-bottomed cell culture plate at 2.5 × 10⁵ cell/well, 60 µL/well. Then diluted anti-ST2 antibodies (serially diluted 3-fold from an initial concentration of 53 nM) were added at 15 µL/well. The plate was incubated at 37 °C for 30 min. After the incubation, 15 µL of hIL33 protein (with a final concentration of 4 ng/mL) and 15 µL of IL2 (with a final concentration of 10 ng/mL) (PrimeGene, GMP-101-02) were added to each well. The mixtures were well mixed and incubated at 37 °C for 48 h. After the incubation, the supernatants were collected and assayed for the IL5 content by following the instructions in the IL5 assay kit (R&D, DY205). The OD450 values were read using a microplate reader (PE, Envision). The data were analyzed using GraphPad Prism5, and the IC₅₀ values were calculated. The IC₅₀ results for the anti-ST2 chimeric antibodies' blocking of the IL33/ST2-induced IL5 secretion on CD4⁺ T cells are shown in Table 10.

**Table 10. The IC₅₀ values for the anti-ST2 chimeric antibodies' blocking of the IL33/ST2-induced IL5 secretion on CD4⁺ T cells**

| Antibody | IC50(nM) |
|---|---|
| RG6149 | 0.320 |
| xi7D1 | 0.120 |
| xi3C6 | ND |
| xi31C8 | 0.090 |
| xi26A1 | ND |
| xi8F4 | ND |
| xi17E2 | 0.080 |

| | |
|---|---|
| "ND" indicates "not detected". | |

### Example 11. Humanization of Anti-Human ST2 Hybridoma Monoclonal Antibodies

31C8 (the amino acid sequence of the heavy chain variable region set forth in SEQ ID NO: 39 and the amino acid sequence of the light chain variable region set forth in SEQ ID NO: 40) was selected for humanization design. The established CDR-grafting method was adopted, and a human antibody framework region that could be used for humanization of the mouse antibody were selected. A human germline antibody or a subtype thereof with the highest sequence identity (i.e., sequence similarity) to the amino acids in the mouse antibody variable region were selected. The heavy chain variable region CDRs and the light chain variable region CDRs of the mouse antibody were inserted into the selected framework region, and the residues in the framework region were mutated. Some CDRs were mutated further to improve the properties of the antibody, such as the physicochemical properties or druggability. Humanized antibodies hz31C8-1.1 and hz31C8-1.2 were obtained. The amino acid sequences of the heavy chain variable region and the light chain variable region of hz31C8-1.1 were SEQ ID NO: 71 and SEQ ID NO: 79, respectively. The DNA sequences encoding the heavy chain variable region and the light chain variable region of the humanized antibody hz31C8-1.1 were SEQ ID NO: 72 and SEQ ID NO: 80, respectively. The amino acid sequences of the heavy chain variable region and the light chain variable region of hz31C8-1.2 were SEQ ID NO: 73 and SEQ ID NO: 79, respectively. The DNA sequences encoding the heavy chain variable region and the light chain variable region of the humanized antibody hz31C8-1.2 were SEQ ID NO: 74 and SEQ ID NO: 80, respectively.

The humanized VL region gene synthetic fragment was ligated to the human κ chain constant region by double digestion to construct a humanized light chain, and the humanized VH region gene synthetic fragment was ligated to the human IgG2 constant region by double digestion to construct a humanized heavy chain. Expression vectors were transfected with the humanized heavy chain and light chain DNA corresponding to each antibody. Protein expression was performed using the ExpiCHO expression system. Then the humanized antibodies in the cell culture supernatants were purified through a Protein A column.

### Example 12. Biological Function Assays of Humanized Anti-ST2 Antibodies

The humanized antibodies hz31C81-1.1 (the amino acid sequence of the heavy chain set forth in SEQ ID NO: 75 and the amino acid sequence of the light chain set forth in SEQ ID NO: 81) and hz31C8-1.2 ( the amino acid sequence of the heavy chain set forth in SEQ ID NO: 77 and the amino acid sequence of the light chain set forth in SEQ ID NO: 81) were subjected to affinity, binding, blocking and cell function analyses using the methods described in Example 4, Example 5, Example 6, Example 7, Example 8, Example 9 and Example 10. In the present disclosure, "hz" and "xi" denote humanized antibodies and chimeric antibodies, respectively; for example, "hz31C81-1.1" and "hz31C81-1.2" represent humanized 31C81-1.1 and 31C81-1.2 antibodies, and "xi31C8" represents a chimeric 31C8 antibody.

The affinity of the anti-ST2 humanized antibodies for ST2 is shown in Table 11. The EC₅₀ values for the binding of the anti-ST2 humanized antibodies to hST2 and Cyno-ST2 in the ELISA assays are shown in Table 12 and FIGs. 4-5. The IC₅₀ values for the anti-ST2 humanized antibodies' blocking of IL33/ST2 binding in the ELISA assays are shown in Table 13 and FIG. 6. The EC₅₀ values for the binding of the anti-ST2 humanized antibodies to hST2 at the cellular level are shown in Table 14 and FIG. 10. The IC₅₀ values for the anti-ST2 humanized antibodies' blocking of IL33/ST2 signaling at the cellular level are shown in Table 15 and FIGs. 11-12. The IC₅₀ values for the anti-ST2 humanized antibodies' blocking of the IL33/ST2-induced IL5 secretion on CD4⁺ T cells are shown in Table 16 and FIG. 13.

**Table 11. The affinity of the anti-ST2 humanized antibodies for ST2**

| Antibody | KD(M) | |
|---|---|---|
| | hST2 | Cyno-ST2 |
| GSK3772847 | 9.58E-11 | 9.23E-11 |
| RG6149 | 1.28E-10 | 6.90E-11 |
| xi31C8 | 1.29E-10 | 1.62E-09 |
| hz31C8-1.1 | 8.36E-11 | 2.47E-09 |
| hz31C8-1.2 | 1.45E-10 | 5.27E-09 |

**Table 12. The EC₅₀ values for the binding of the anti-ST2 humanized antibodies to hST2 and Cyno-ST2 in the ELISA assays**

| Antibody | EC50(nM) | |
|---|---|---|
| | hST2 | Cyno-ST2 |
| GSK3772847 | 0.1266 | 0.2418 |
| RG6149 | 0.1125 | 0.1778 |
| xi31C8 | 0.0865 | 0.0690 |
| hz31C8-1.1 | 0.0879 | 0.0716 |
| hz31C8-1.2 | 0.0937 | 0.0746 |

**Table 13. The IC₅₀ values for the anti-ST2 humanized antibodies' blocking of IL33/ST2 binding in the ELISA assays**

| Antibody | IC50(nM) |
|---|---|
| GSK3772847 | 2.533 |
| RG6149 | 2.151 |
| xi31C8 | 1.933 |
| hz31C8-1.1 | 1.893 |
| hz31C8-1.2 | 2.241 |

**Table 14. The EC₅₀ values for the binding of the anti-ST2 humanized antibodies to hST2 at the cellular level**

| Antibody | EC50(nM) |
|---|---|
| | HEK293T-hST2-NFxB-Luciferase |
| GSK3772847 | 0.6756 |
| RG6149 | 0.5814 |
| xi31C8 | 0.4049 |
| hz31C8-1.1 | 0.3844 |
| hz31C8-1.2 | 0.4676 |

**Table 15. The IC₅₀ values for the anti-ST2 humanized antibodies' blocking of the IL33/ST2 signaling at the cellular level**

| Antibody | IC50(nM) | |
|---|---|---|
| | HEK293T-hST2-NFxB-Luciferase | KU812-NFxB-Luciferase |
| GSK3772847 | 29.160 | 0.111 |
| RG6149 | 84.280 | 0.047 |
| xi31C8 | 15.630 | 0.016 |
| hz31C8-1.1 | 15.950 | 0.008 |
| hz31C8-1.2 | 20.370 | 0.006 |

**Table 16. The IC₅₀ values for the anti-ST2 humanized antibodies' blocking of the IL33/ST2-induced IL5 secretion on CD4⁺ T cells**

| Antibody | IC50(nM) |
|---|---|
| GSK3772847 | 0.794 |
| RG6149 | 1.819 |
| xi31C8 | 0.440 |
| hz31C8-1.1 | 0.462 |
| hz31C8-1.2 | 0.366 |

### Example 13. Stability Assays of Humanized Anti-ST2 Antibodies

The thermostability of the different antibodies was determined by DSC (differential scanning calorimetry). Samples were dissolved in PBS buffer (pH 7.4) and determination was performed using MicroCal VP-Capillary DSC (Malvern Panalytical). As shown in Table 17, the humanized anti-ST2 antibodies hz31C8-1.1 and hz31C8-1.2 of the present disclosure both showed good thermostability and were superior to the control anti-ST2 antibodies RG6149 and GSK3772847.

**Table 17. The thermostability of the antibodies determined by DSC**

| Antibody | Tm initial (°C) | Tm1 (°C) | Tm2 (°C) | Tm3 (°C) |
|---|---|---|---|---|
| hz31C8-1.1 | 63.12 | 80.12 | / | / |
| hz31C8-1.2 | 63.84 | 77.92 | / | / |
| RG6149 | 57.47 | 64.51 | 76.94 | / |
| GSK3772847 | 57.24 | 64.70 | 71.32 | 76.29 |

The periodic stability of the antibodies was determined by SEC-HPLC at certain concentrations, for example, the antibody concentration was controlled at about 1 mg/mL, and stored in PBS buffer (pH 7.2) at, e.g., 40 °C for 2 weeks. The determination was performed using LC-20ADXR/DGU (Shimadzu). As shown below in Table 18, the humanized anti-ST2 antibodies hz31C8-1.1 and hz31C8-1.1 of the present disclosure both showed good stability.

**Table 18. The periodic stability of the antibodies determined by SEC-HPLC**

| Antibody | Purity (main area%) | Aggregate content (area%) | Fragment content (area%) |
|---|---|---|---|
| xi31C8 | 96.41 | 3.59 | 0 |
| hz31C8-1.1 | 94.30 | 5.70 | 0 |
| hz31C8-1.2 | 99.67 | 0.33 | 0 |
| RG6149 | 93.21 | 6.79 | 0 |
| GSK3772847 | 79.29 | 10.11 | 10.60 |

The anti-ST2 antibodies were diluted with PBS buffer to a concentration of 1.5 mg/mL. After being treated at 72 °C for 5 min, the samples were assayed by ELISA for their binding activity for an antigen. The ELISA procedure is as follows: A 96-well plate was coated with 2 µg/mL hST2-ECD-mFc as the antigen at 100 µL/well and incubated at 4 °C overnight. After the antigen not adsorbed was well washed off, the non-specific binding sites were blocked with a blocking buffer (PBST containing 1% bovine serum albumin). After the plate was washed three times with a washing buffer (PBST, PBS containing 0.05% (v/v) Tween 20), the anti-ST2 antibodies (serially diluted 3-fold from an initial concentration of 1.5 µg/mL to 8 concentrations) were added at 100 µL/well, and the plate was incubated at 37 °C for 1 h. After the plate was washed with the washing buffer, the anti-Human IgG Fcy secondary antibody (Jackson ImmunoResearch, 109-035-008) was added, and the plate was incubated at 37 °C for 1 h. After the plate was washed with the washing buffer, the substrate TMB solution (Thermo, 00-4201-56) was added at 100 µL/well for color development. After the plate was incubated at room temperature for 5 min, a stop solution (2 N H₂SO₄) was added to stop the reactions. The signals were read at 450 nm using a plate reader (PE, Envision). The data were analyzed using GraphPad Prism5, and the EC₅₀ values were calculated. As shown below in Table 19, the humanized anti-ST2 antibodies hz31C8-1.1 and hz31C8-1.1 of the present disclosure both showed good thermostability.

**Table 19. The binding activity of the heated antibodies determined by ELISA**

| Antibody | EC50(nM) | |
|---|---|---|
| | Not heated | Heated |
| RG6149 | 0.1428 | 1.064 |
| GSK3772847 | 0.1089 | 0.1877 |
| xi31C8 | 0.0744 | 0.0863 |
| hz31C8-1.1 | 0.0906 | 0.0968 |
| hz31C8-1.2 | 0.0920 | 0.1095 |

### Example 14. Cross-Reactivity of Humanized Anti-ST2 Antibodies with IL1R Family Receptors

ST2 belongs to the IL1R receptor family. The cross-reactivity of the anti-ST2 antibodies with the IL1R family receptors was determined by ELISA. The IL1R family receptors include IL1R1/CD121a (Sino biological, 10126-H08H), IL1R2/CD121b (Sino biological, 10111-H08H), IL1R3/IL1RAP (Sino biological, 10121-H08H), IL1R7/IL-18RAcP (Sino biological, 10176-H08H), IL1R8/IL1RAPL1 (Sino biological, 10177-H08H), and IL1R9/IL1RAPL2 (Sino biological, 10156-H08H). ELISA assays were performed using the proteins described above as antigens (2 µg/mL). See Example 13 for the specific ELISA procedure. No binding means that the antibody remains unbound to the antigen when at the highest concentration. Weak binding means that the antibody binds weakly to the antigen when at the highest concentration and does not bind when at low concentrations. Strong binding means that a very good binding curve could be fit to the binding data.

**Table 20. The cross-reactivity of the humanized ST2 antibodies with the IL1R family receptors determined by ELISA**

| IL1R family | Cross reaction | | | | |
|---|---|---|---|---|---|
| | hz31C8-1.1 | hz31C8-1.2 | xi31C8 | GSK3772847 | RG6149 |
| IL1R1/CD121a | - | - | - | - | - |
| IL1R2/CD121b | - | - | - | + | - |
| IL1R3/IL1RAP | - | - | - | - | - |
| IL1R7/IL-18RAcP | - | - | - | + | - |
| IL1R8/IL1RAPL1 | - | - | - | - | - |
| IL1R9/ IL1RAPL2 | - | - | - | - | - |
| ST2 | +++++ | +++++ | +++++ | +++++ | +++++ |

| | | | | | |
|---|---|---|---|---|---|
| (-: no binding; +: weak binding; +++++: strong binding) | | | | | |

### Example 15. Pharmacokinetic Evaluation of Humanized Anti-ST2 Antibodies

Eighteen laboratory Balb/c mice, divided into groups of 6, under 12/12-hour visual adjustment, given *ad libitum* access to food and water, purchased from the Model Animal Research Center of Nanjing University. On the day of experiment, the mice were each dosed with a 1 mg/mL sample by intravenous injection at 10 mg/kg. The orbital blood was collected before the dosing (0 min, or one day or longer before the dosing), and 30 min, 8 h, 24 h, 2 d (48 h), 4 d (96 h), 7 d, and 14 d after the dosing, and then the serum was collected and assayed for the antibody concentration by ELISA. The ELISA procedure is as follows: A 96-well plate was coated with 2 µg/mL ST2-His (sinobiological, 10105-H08H) as the antigen at 100 µL/well and incubated at 4 °C overnight. After the antigen not adsorbed was well washed off, the non-specific binding sites were blocked with a blocking buffer (PBST containing 1% bovine serum albumin). After the plate was washed three times with a washing buffer (PBST, PBS containing 0.05% (v/v) Tween 20), 100 µL of the test serum was added, and the plate was incubated at 37 °C for 1 h. After the plate was washed with the washing buffer, the anti-Mouse IgG Fcy secondary antibody was added, and the plate was incubated at 37 °C for 1 h. After the plate was washed with the washing buffer, the substrate TMB solution (Thermo, 00-4201-56) was added at 100 µL/well for color development. After the plate was incubated at room temperature for 5 min, a stop solution (2 N H₂SO₄) was added to stop the reactions. The signals were read at 450 nm using a plate reader (PE, Envision). The data were analyzed using GraphPad PrismS. Referring to Table 21, the PK analysis shows that the humanized anti-ST2 antibodies hz31C8-1.1 and hz31C8-1.1 of the present disclosure have half-lives of about 9.7 d and 7.33 d, superior to the control anti-ST2 antibody RG6149.

**Table 21. The T 1/2 of the humanized ST2 antibodies in mice**

| Antibody | T1/2 (mean ± SD, d) |
|---|---|
| RG6149 | 4.61±2.40 |
| hz31C8-1.1 | 9.70±3.11 |
| hz31C8-1.2 | 7.33±4.37 |

Although the present disclosure has been described in detail herein above through general explanations and specific embodiments, it will be apparent to those skilled in the art that modifications or improvements can be made based on the present disclosure. Accordingly, such modifications and improvements made without departing from the spirit of the present disclosure all fall within the protection scope of the present disclosure.

## Claims

1. An isolated ST2 antibody or an antigen-binding fragment thereof, wherein the antibody or the antigen-binding fragment thereof comprises:
(i) heavy chain CDR1, heavy chain CDR2 and heavy chain CDR3, wherein
(1) heavy chain CDR1 comprises a sequence set forth in SEQ ID NO: 11 or an amino acid sequence having at least 80% identity thereto, heavy chain CDR2 comprises a sequence set forth in SEQ ID NO: 15 or an amino acid sequence having at least 80% identity thereto, and heavy chain CDR3 comprises a sequence set forth in SEQ ID NO: 20 or an amino acid sequence having at least 80% identity thereto;
(2) heavy chain CDR1 comprises a sequence set forth in SEQ ID NO: 11 or an amino acid sequence having at least 80% identity thereto, heavy chain CDR2 comprises a sequence set forth in SEQ ID NO: 16 or an amino acid sequence having at least 80% identity thereto, and heavy chain CDR3 comprises a sequence set forth in SEQ ID NO: 21 or an amino acid sequence having at least 80% identity thereto;
(3) heavy chain CDR1 comprises a sequence set forth in SEQ ID NO: 12 or an amino acid sequence having at least 80% identity thereto, heavy chain CDR2 comprises a sequence set forth in SEQ ID NO: 17 or an amino acid sequence having at least 80% identity thereto, and heavy chain CDR3 comprises a sequence set forth in SEQ ID NO: 22 or an amino acid sequence having at least 80% identity thereto;
(4) heavy chain CDR1 comprises a sequence set forth in SEQ ID NO: 13 or an amino acid sequence having at least 80% identity thereto, heavy chain CDR2 comprises a sequence set forth in SEQ ID NO: 18 or an amino acid sequence having at least 80% identity thereto, and heavy chain CDR3 comprises a sequence set forth in SEQ ID NO: 23 or an amino acid sequence having at least 80% identity thereto;
(5) heavy chain CDR1 comprises a sequence set forth in SEQ ID NO: 14 or an amino acid sequence having at least 80% identity thereto, heavy chain CDR2 comprises a sequence set forth in SEQ ID NO: 19 or an amino acid sequence having at least 80% identity thereto, and heavy chain CDR3 comprises a sequence set forth in SEQ ID NO: 22 or an amino acid sequence having at least 80% identity thereto; or
(6) heavy chain CDR1 comprises a sequence set forth in SEQ ID NO: 11 or an amino acid sequence having at least 80% identity thereto, heavy chain CDR2 comprises a sequence set forth in SEQ ID NO: 18 or an amino acid sequence having at least 80% identity thereto, and heavy chain CDR3 comprises a sequence set forth in SEQ ID NO: 24 or an amino acid sequence having at least 80% identity thereto;
(ii) light chain CDR1, light chain CDR2 and light chain CDR3, wherein
(1) light chain CDR1 comprises a sequence set forth in SEQ ID NO: 25 or an amino acid sequence having at least 80% identity thereto, light chain CDR2 comprises a sequence set forth in SEQ ID NO: 29 or an amino acid sequence having at least 80% identity thereto, and light chain CDR3 comprises a sequence set forth in SEQ ID NO: 32 or an amino acid sequence having at least 80% identity thereto;
(2) light chain CDR1 comprises a sequence set forth in SEQ ID NO: 26 or an amino acid sequence having at least 80% identity thereto, light chain CDR2 comprises a sequence set forth in SEQ ID NO: 30 or an amino acid sequence having at least 80% identity thereto, and light chain CDR3 comprises a sequence set forth in SEQ ID NO: 34 or an amino acid sequence having at least 80% identity thereto;
(3) light chain CDR1 comprises a sequence set forth in SEQ ID NO: 27 or an amino acid sequence having at least 80% identity thereto, light chain CDR2 comprises a sequence set forth in SEQ ID NO: 30 or an amino acid sequence having at least 80% identity thereto, and light chain CDR3 comprises a sequence set forth in SEQ ID NO: 34 or an amino acid sequence having at least 80% identity thereto; or
(4) light chain CDR1 comprises a sequence set forth in SEQ ID NO: 28 or an amino acid sequence having at least 80% identity thereto, light chain CDR2 comprises a sequence set forth in SEQ ID NO: 31 or an amino acid sequence having at least 80% identity thereto, and light chain CDR3 comprises a sequence set forth in SEQ ID NO: 33 or an amino acid sequence having at least 80% identity thereto; or
(iii) heavy chain CDR1, heavy chain CDR2 and heavy chain CDR3 set forth in (i), and light chain CDR1, light chain CDR2 and light chain CDR3 set forth in (ii), wherein
the amino acid sequence of SEQ ID NO: 17 is AIDPETGDTVYX₁X₂KFX₃G, wherein X₁ = N or A, X₂ = Q, E or K, and X₃ = K or Q;
the amino acid sequence of SEQ ID NO: 30 is QX₄SNLAS, wherein X₄ = M or L.

2. The antibody or the antigen-binding fragment thereof according to claim 1, wherein
the amino acid sequence of SEQ ID NO: 17 is AIDPETGDTVYX₁X₂KFX₃G, wherein
(i) X₁ = N or A, X₂ = Q, and X₃ = K or Q;
(ii) X₁ = N or A, X₂ = E, and X₃ = K or Q; or
(iii) X₁ = N or A, X₂ = K, X₃ = K or Q.

3. The antibody or the antigen-binding fragment thereof according to claim 1 or 2, wherein the antibody or the antigen-binding fragment thereof comprises heavy chain CDR1, heavy chain CDR2, heavy chain CDR3, light chain CDR1, light chain CDR2, and light chain CDR3, wherein
(1) heavy chain CDR1 comprises a sequence set forth in SEQ ID NO: 11 or an amino acid sequence having at least 80% identity thereto, heavy chain CDR2 comprises a sequence set forth in SEQ ID NO: 15 or an amino acid sequence having at least 80% identity thereto, heavy chain CDR3 comprises a sequence set forth in SEQ ID NO: 20 or an amino acid sequence having at least 80% identity thereto, light chain CDR1 comprises a sequence set forth in SEQ ID NO: 25 or an amino acid sequence having at least 80% identity thereto, light chain CDR2 comprises a sequence set forth in SEQ ID NO: 29 or an amino acid sequence having at least 80% identity thereto, and light chain CDR3 comprises a sequence set forth in SEQ ID NO: 32 or an amino acid sequence having at least 80% identity thereto;
(2) heavy chain CDR1 comprises a sequence set forth in SEQ ID NO: 11 or an amino acid sequence having at least 80% identity thereto, heavy chain CDR2 comprises a sequence set forth in SEQ ID NO: 16 or an amino acid sequence having at least 80% identity thereto, heavy chain CDR3 comprises a sequence set forth in SEQ ID NO: 21 or an amino acid sequence having at least 80% identity thereto, light chain CDR1 comprises a sequence set forth in SEQ ID NO: 26 or an amino acid sequence having at least 80% identity thereto, light chain CDR2 comprises a sequence set forth in SEQ ID NO: 30 or an amino acid sequence having at least 80% identity thereto, and light chain CDR3 comprises a sequence set forth in SEQ ID NO: 34 or an amino acid sequence having at least 80% identity thereto;
(3) heavy chain CDR1 comprises a sequence set forth in SEQ ID NO: 12 or an amino acid sequence having at least 80% identity thereto, heavy chain CDR2 comprises a sequence set forth in SEQ ID NO: 17 or an amino acid sequence having at least 80% identity thereto, heavy chain CDR3 comprises a sequence set forth in SEQ ID NO: 22 or an amino acid sequence having at least 80% identity thereto, light chain CDR1 comprises a sequence set forth in SEQ ID NO: 27 or an amino acid sequence having at least 80% identity thereto, light chain CDR2 comprises a sequence set forth in SEQ ID NO: 30 or an amino acid sequence having at least 80% identity thereto, and light chain CDR3 comprises a sequence set forth in SEQ ID NO: 34 or an amino acid sequence having at least 80% identity thereto;
(4) heavy chain CDR1 comprises a sequence set forth in SEQ ID NO: 13 or an amino acid sequence having at least 80% identity thereto, heavy chain CDR2 comprises a sequence set forth in SEQ ID NO: 18 or an amino acid sequence having at least 80% identity thereto, heavy chain CDR3 comprises a sequence set forth in SEQ ID NO: 23 or an amino acid sequence having at least 80% identity thereto, light chain CDR1 comprises a sequence set forth in SEQ ID NO: 27 or an amino acid sequence having at least 80% identity thereto, light chain CDR2 comprises a sequence set forth in SEQ ID NO: 30 or an amino acid sequence having at least 80% identity thereto, and light chain CDR3 comprises a sequence set forth in SEQ ID NO: 34 or an amino acid sequence having at least 80% identity thereto;
(5) heavy chain CDR1 comprises a sequence set forth in SEQ ID NO: 14 or an amino acid sequence having at least 80% identity thereto, heavy chain CDR2 comprises a sequence set forth in SEQ ID NO: 19 or an amino acid sequence having at least 80% identity thereto, heavy chain CDR3 comprises a sequence set forth in SEQ ID NO: 22 or an amino acid sequence having at least 80% identity thereto, light chain CDR1 comprises a sequence set forth in SEQ ID NO: 27 or an amino acid sequence having at least 80% identity thereto, light chain CDR2 comprises a sequence set forth in SEQ ID NO: 30 or an amino acid sequence having at least 80% identity thereto, and light chain CDR3 comprises a sequence set forth in SEQ ID NO: 34 or an amino acid sequence having at least 80% identity thereto; or
(6) heavy chain CDR1 comprises a sequence set forth in SEQ ID NO: 11 or an amino acid sequence having at least 80% identity thereto, heavy chain CDR2 comprises a sequence set forth in SEQ ID NO: 18 or an amino acid sequence having at least 80% identity thereto, heavy chain CDR3 comprises a sequence set forth in SEQ ID NO: 24 or an amino acid sequence having at least 80% identity thereto, light chain CDR1 comprises a sequence set forth in SEQ ID NO: 28 or an amino acid sequence having at least 80% identity thereto, light chain CDR2 comprises a sequence set forth in SEQ ID NO: 31 or an amino acid sequence having at least 80% identity thereto, and light chain CDR3 comprises a sequence set forth in SEQ ID NO: 33 or an amino acid sequence having at least 80% identity thereto; wherein
the amino acid sequence of SEQ ID NO: 17 is AIDPETGDTVYX₁X₂KFX₃G, wherein
(i) X₁ = N or A, X₂ = Q, and X₃ = K or Q;
(ii) X₁ = N or A, X₂ = E, and X₃ = K or Q; or
(iii) X₁ = N or A, X₂ = K, X₃ = K or Q;
in addition, the amino acid sequence of SEQ ID NO: 30 is QX₄SNLAS, wherein X₄ = M or L.

4. The antibody or the antigen-binding fragment thereof according to any one of claims 1 to 3, wherein the antibody or the antigen-binding fragment thereof is chimeric or humanized.

5. An isolated ST2 antibody or an antigen-binding fragment thereof, wherein the antibody or the antigen-binding fragment thereof comprises:
(i) a heavy chain variable region, wherein the amino acid sequence of the heavy chain variable region comprises a sequence set forth in SEQ ID NO: 35, 37, 39, 41, 43, 45, 71 or 73, or an amino acid sequence having at least 80% identity to the sequence set forth in SEQ ID NO: 35, 37, 39, 41, 43, 45, 71 or 73;
(i) a light chain variable region, wherein the amino acid sequence of the light chain variable region comprises a sequence set forth in SEQ ID NO: 36, 38, 40, 42, 44, 46 or 79, or an amino acid sequence having at least 80% identity to the sequence set forth in SEQ ID NO: 36, 38, 40, 42, 44, 46 or 79; or
(iii) a heavy chain variable region set forth in (i) and a light chain variable region set forth in (ii).

6. The antibody or the antigen-binding fragment thereof according to claim 5, wherein the antibody or the antigen-binding fragment thereof comprises a heavy chain variable region and a light chain variable region, wherein
(1) the heavy chain variable region comprises a sequence set forth in SEQ ID NO: 35 or an amino acid sequence having at least 80% identity thereto, and the light chain variable region comprises a sequence set forth in SEQ ID NO: 36 or an amino acid sequence having at least 80% identity thereto;
(2) the heavy chain variable region comprises a sequence set forth in SEQ ID NO: 37 or an amino acid sequence having at least 80% identity thereto, and the light chain variable region comprises a sequence set forth in SEQ ID NO: 38 or an amino acid sequence having at least 80% identity thereto;
(3) the heavy chain variable region comprises a sequence set forth in SEQ ID NO: 39 or an amino acid sequence having at least 80% identity thereto, and the light chain variable region comprises a sequence set forth in SEQ ID NO: 40 or an amino acid sequence having at least 80% identity thereto;
(4) the heavy chain variable region comprises a sequence set forth in SEQ ID NO: 41 or an amino acid sequence having at least 80% identity thereto, and the light chain variable region comprises a sequence set forth in SEQ ID NO: 42 or an amino acid sequence having at least 80% identity thereto;
(5) the heavy chain variable region comprises a sequence set forth in SEQ ID NO: 43 or an amino acid sequence having at least 80% identity thereto, and the light chain variable region comprises a sequence set forth in SEQ ID NO: 44 or an amino acid sequence having at least 80% identity thereto;
(6) the heavy chain variable region comprises a sequence set forth in SEQ ID NO: 45 or an amino acid sequence having at least 80% identity thereto, and the light chain variable region comprises a sequence set forth in SEQ ID NO: 46 or an amino acid sequence having at least 80% identity thereto;
(7) the heavy chain variable region comprises a sequence set forth in SEQ ID NO: 71 or an amino acid sequence having at least 80% identity thereto, and the light chain variable region comprises a sequence set forth in SEQ ID NO: 79 or an amino acid sequence having at least 80% identity thereto; or
(8) the heavy chain variable region comprises a sequence set forth in SEQ ID NO: 73 or an amino acid sequence having at least 80% identity thereto, and the light chain variable region comprises a sequence set forth in SEQ ID NO: 79 or an amino acid sequence having at least 80% identity thereto.

7. An isolated ST2 antibody or an antigen-binding fragment thereof, wherein the antibody or the antigen-binding fragment thereof comprises:
(i) a heavy chain, wherein the amino acid sequence of the heavy chain comprises a sequence set forth in SEQ ID NO: 47, 51, 55, 59, 63, 67, 75 or 77, or an amino acid sequence having at least 80% identity to the sequence set forth in SEQ ID NO: 47, 51, 55, 59, 63, 67, 75 or 77;
(ii) a light chain, wherein the amino acid sequence of the light chain comprises a sequence set forth in SEQ ID NO: 49, 53, 57, 61, 65, 69 or 81, or an amino acid sequence having at least 80% identity to the sequence set forth in SEQ ID NO: 49, 53, 57, 61, 65, 69 or 81; or
(iii) a heavy chain set forth in (i) and a light chain set forth in (ii).

8. The antibody or the antigen-binding fragment thereof according to claim 7, wherein the antibody or the antigen-binding fragment thereof comprises a heavy chain and a light chain, wherein
(1) the heavy chain comprises a sequence set forth in SEQ ID NO: 47 or an amino acid sequence having at least 80% identity thereto, and the light chain comprises a sequence set forth in SEQ ID NO: 49 or an amino acid sequence having at least 80% identity thereto;
(2) the heavy chain comprises a sequence set forth in SEQ ID NO: 51 or an amino acid sequence having at least 80% identity thereto, and the light chain comprises a sequence set forth in SEQ ID NO: 53 or an amino acid sequence having at least 80% identity thereto;
(3) the heavy chain comprises a sequence set forth in SEQ ID NO: 55 or an amino acid sequence having at least 80% identity thereto, and the light chain comprises a sequence set forth in SEQ ID NO: 57 or an amino acid sequence having at least 80% identity thereto;
(4) the heavy chain comprises a sequence set forth in SEQ ID NO: 59 or an amino acid sequence having at least 80% identity thereto, and the light chain comprises a sequence set forth in SEQ ID NO: 61 or an amino acid sequence having at least 80% identity thereto;
(5) the heavy chain comprises a sequence set forth in SEQ ID NO: 63 or an amino acid sequence having at least 80% identity thereto, and the light chain comprises a sequence set forth in SEQ ID NO: 65 or an amino acid sequence having at least 80% identity thereto;
(6) the heavy chain comprises a sequence set forth in SEQ ID NO: 67 or an amino acid sequence having at least 80% identity thereto, and the light chain comprises a sequence set forth in SEQ ID NO: 69 or an amino acid sequence having at least 80% identity thereto;
(7) the heavy chain comprises a sequence set forth in SEQ ID NO: 75 or an amino acid sequence having at least 80% identity thereto, and the light chain comprises a sequence set forth in SEQ ID NO: 81 or an amino acid sequence having at least 80% identity thereto; or
(8) the heavy chain comprises a sequence set forth in SEQ ID NO: 77 or an amino acid sequence having at least 80% identity thereto, and the light chain comprises a sequence set forth in SEQ ID NO: 81 or an amino acid sequence having at least 80% identity thereto.

9. The antibody or the antigen-binding fragment thereof according to any one of claims 1 to 8, wherein the antibody or the antigen-binding fragment thereof is selected from a monoclonal antibody, an Fab fragment, an F(ab')2 fragment, an Fd fragment, an Fv fragment, a dAb, an isolated CDR region, a single-chain Fv molecule, or combinations thereof.

10. The antibody or the antigen-binding fragment thereof according to any one of claims 1 to 9, wherein the antibody or the antigen-binding fragment thereof specifically binds to human or monkey ST2 and blocks binding of IL33/ST2 and signaling thereof.

11. The antibody or the antigen-binding fragment thereof according to any one of claims 1 to 10, wherein the antibody or the antigen-binding fragment thereof does not substantially bind to IL1R1, IL1R2, IL1R3, IL1R7, IL1R8 and IL1R9.

12. The antibody or the antigen-binding fragment thereof according to any one of claims 1 to 11, wherein the antibody or the antigen-binding fragment thereof blocks IL5 secretion induced by IL33/ST2 on CD4⁺ T cells.

13. An isolated nucleic acid molecule encoding the antibody or the antigen-binding fragment thereof according to any one of claims 1 to 12.

14. An expression vector comprising the nucleic acid molecule according to claim 13.

15. A host cell comprising the nucleic acid molecule according to claim 13 or the expression vector according to claim 14.

16. A polypeptide fusion or multispecific molecule comprising the antibody or the antigen-binding fragment thereof according to any one of claims 1 to 12.

17. A viral vector comprising a nucleic acid molecule encoding the antibody or the antigen-binding fragment thereof according to any one of claims 1 to 12 or the nucleic acid molecule according to claim 13.

18. A pharmaceutical composition comprising the antibody or the antigen-binding fragment thereof according to any one of claims 1 to 12, and one or more pharmaceutically acceptable excipients, diluents or carriers.

19. A method for determining an ST2 expression level in a sample from a subject, wherein the method comprises contacting the sample with the antibody or the antigen-binding fragment thereof according to any one of claims 1 to 12 under such conditions that the antibody or the antigen-binding fragment thereof and ST2 form a complex.

20. A method for use in treating or ameliorating IL33/ST2-mediated related diseases and conditions in a subject in need, wherein the method comprises administering to the subject a therapeutically effective amount of the antibody or the antigen-binding fragment thereof according to any one of claims 1 to 12 or the nucleic acid molecule according to claim 13.

21. The method according to claim 20, wherein the IL33/ST2-mediated related diseases and conditions include asthma, allergic rhinitis, chronic obstructive pulmonary disease, eosinophilic bronchitis, eosinophilic esophagitis, atopic dermatitis, psoriasis, systemic lupus erythematosus, pemphigoid, rheumatoid arthritis, ankylosing spondylitis, inflammatory bowel disease, pulmonary fibrosis, hepatic fibrosis, systemic sclerosis, sarcoidosis, graft-versus-host disease (GVHD), diabetes, cardiovascular diseases, or combinations thereof.
